# EUROPEAN PATENT SPECIFICATION

(11) **EP 4 226 856 B1**
(45) Date of publication and mention of the grant of the patent: **09.12.2026**
(21) Application number: 21878943.6
(22) Date of filing: 19.04.2021
(51) Int. Cl.: A61B 5/021, A61B 5/00, A61B 5/024, G06N 3/02, G06N 3/09, G06N 3/045, G06N 3/048, G06N 3/0464

(54) **METHOD AND APPARATUS FOR PREDICTING BLOOD PRESSURE BY FUSING CALIBRATED PHOTOPLETHYSMOGRAPHIC SIGNAL DATA**
VERFAHREN UND VORRICHTUNG ZUR VORHERSAGE DES BLUTDRUCKS DURCH FUSION KALIBRIERTER PHOTOPLETHYSMOGRAPHISCHER SIGNALDATEN
PROCÉDÉ ET APPAREIL DE PRÉDICTION DE LA PRESSION SANGUINE PAR FUSION DE DONNÉES DE SIGNAL PHOTOPLÉTHYSMOGRAPHIQUE ÉTALONNÉES

(30) Priority: 12.10.2020 CN 202011085575
(43) Date of publication of application: 16.08.2023
(73) Proprietor: Lepu Medical Technology (Beijing) Co., Ltd., Beijing 102200 (CN)
(72) Inventor: CAO, Jun, Beijing 102200 (CN); ZHANG, Biying, Beijing 102200 (CN); WANG, Sihan, Beijing 102200 (CN); WU, Zejian, Beijing 102200 (CN)
(74) Representative: Glück Kritzenberger Patentanwälte PartGmbB
(86) International application number: PCT/CN2021/088023
(87) International publication number: WO 2022/077889

(56) References cited:
- EP-A1- 2 544 125
- CN-A- 104 665 768
- CN-A- 109 452 935
- CN-A- 111 063 420
- CN-A- 111 248 882
- CN-A- 111 248 883
- CN-A- 111 248 883
- CN-A- 111 291 727
- CN-A- 111 291 727
- CN-A- 111 358 451
- CN-A- 111 358 453
- CN-A- 112 336 325
- US-A1- 2020 093 386
- SCHLESINGER ODED ET AL: "Estimation and Tracking of Blood Pressure Using Routinely Acquired Photoplethysmographic Signals and Deep Neural Networks", CRITICAL CARE EXPLORATIONS, vol. 2, no. 4, 8 April 2020 (2020-04-08), pages e0095, XP093234365, ISSN: 2639-8028, DOI: 10.1097/CCE.0000000000000095
- SCHLESINGER ODED ET AL: "Blood Pressure Estimation From PPG Signals Using Convolutional Neural Networks And Siamese Network", ICASSP 2020 - 2020 IEEE INTERNATIONAL CONFERENCE ON ACOUSTICS, SPEECH AND SIGNAL PROCESSING (ICASSP), IEEE, 4 May 2020 (2020-05-04), pages 1135 - 1139, XP033793112, DOI: 10.1109/ICASSP40776.2020.9053446
- HAIXIA FAN, XIAOHUI CHEN: "A Continuous Non-invasive Blood Pressure Prediction Method Based on Improved SVR Learning", JOURNAL OF SYSTEM SIMULATION, GAI-KAN BIANJIBU , BEIJING, CN, vol. 32, no. 9, 30 September 2020 (2020-09-30), CN , XP055923421, ISSN: 1004-731X
- SHIMAZAKI SHOTA; KAWANAKA HARUKI; ISHIKAWA HIROKI; INOUE KOICHI; OGURI KOJI: "Cuffless Blood Pressure Estimation from only the Waveform of Photoplethysmography using CNN", 2019 41ST ANNUAL INTERNATIONAL CONFERENCE OF THE IEEE ENGINEERING IN MEDICINE AND BIOLOGY SOCIETY (EMBC), 23 July 2019 (2019-07-23), pages 5042 - 5045, XP033624618, DOI: 10.1109/EMBC.2019.8856706

## Description

This application claims the priority of the China patent application No. 202011085575.X, filed in the National Intellectual Property Administration, PRC on Oct. 12, 2020, and the invention title of "Blood Pressure Prediction Method and Device Fusing Nominal Photoplethysmography (PPG) Signal Data".

### FIELD OF THE INVENTION

The invention relates to the technical field of signal processing, in particular to a blood pressure prediction method and device fusing nominal photoplethysmography (PPG) signal data.

### BACKGROUND OF THE INVENTION

Photoplethysmography (PPG) is a non-invasive method to detect the change of blood volume in viable tissue by photoelectric means. Cardiac impulses make the blood flow per unit area in the blood vessel change periodically, causing blood volume to change accordingly so that a PPG signal indicating the amount of light absorbed by blood will also change periodically, and the periodic changing of the PPG signal is closely related to cardiac impulses and blood pressure change. The diastolic and systolic blood pressure data can be obtained by applying a well-trained artificial intelligence blood pressure prediction network composed of a Convolutional Neural Network (CNN) model and an Artificial Neural Network (ANN) model to the PPG signal.

The publications SCHLESINGER ODED ET AL: "Estimation and Tracking of Blood Pressure Using Routinely Acquired Photoplethysmographic Signals and Deep Neural Networks", CRITICAL CARE EXPLORATIONS, vol. 2, no. 4, 8 April 2020, and SCHLESINGER ODED ET AL: "Blood Pressure Estimation From PPG Signals Using Convolutional Neural Networks And Siamese Network", ICASSP 2020-2020 IEEE INTERNATIONAL CONFERENCE ON ACOUSTICS, SPEECH AND SIGNAL PROCESSING (ICASSP), IEEE, 4 May 2020, pages 1135-1139, both disclose the fusion of nominal (calibration) data and real-time data (current PPG) using two Convolutional Neural Networks and an Artificial Neural Network.

Further methods of predicting blood pressure from PPG measurements using Convolutional Neural Networks are known from documents CN111248883A, CN111248882A and CN111291727A.

### SUMMARY OF THE INVENTION

The purpose of the invention is to provide a blood pressure prediction method and device fusing nominal photoplethysmography (PPG) signal data, electronic equipment, a computer program product and a computer-readable storage medium to overcome the defects of the prior art. By fusing the nominal PPG signal data with real-time PPG signal data, then using a well-trained CNN+ANN prediction network for predicting relative blood pressure data to perform blood pressure prediction on the fused data to obtain the relative blood pressure data, and then performing inverse deduction of absolute blood pressure data according to the relative relationship information which reflecting the relationship between the relative blood pressure data and the nominal blood pressure data to obtain the predicted blood pressure data. In this way, the prediction accuracy of an artificial intelligence blood pressure prediction network can be improved.

In order to achieve the above object, the first aspect of the embodiment of the invention provides a blood pressure prediction method fusing nominal PPG signal data, comprising:
acquiring the nominal PPG signal data and the corresponding nominal diastolic blood pressure data and nominal systolic blood pressure data;
acquiring real-time PPG signal data, a time length of the real-time PPG signal data being the same as that of the nominal PPG signal data;
performing input data preparation processing on a Convolutional Neural Network (CNN) model according to the nominal PPG signal data and the real-time PPG signal data to generate a CNN input four-dimensional tensor;
using the CNN model to perform multi-layer convolution pooling calculation on the CNN input four-dimensional tensor to generate a CNN output four-dimensional tensor;
performing input data preparation processing on an artificial neural network (ANN) model according to the CNN output four-dimensional tensor to generate an ANN input two-dimensional tensor;
using the ANN model to perform regression calculation on the ANN input two-dimensional tensor to generate an ANN output two-dimensional tensor;
performing blood pressure data calculation according to the nominal diastolic blood pressure data, the nominal systolic blood pressure data and the ANN output two-dimensional tensor to generate a blood pressure two-dimensional tensor; and
when preset prediction type information is a first type, performing mean blood pressure data calculation according to the blood pressure two-dimensional tensor to generate diastolic blood pressure prediction data and systolic blood pressure prediction data, and when prediction type information is a second type, performing blood pressure data extraction processing on the blood pressure two-dimensional tensor to generate a diastolic blood pressure prediction data sequence and a systolic blood pressure prediction data sequence,
wherein performing input data preparation processing on a CNN model according to the nominal PPG signal data and the real-time PPG signal data to generate a CNN input four-dimensional tensor specifically comprises:
   according to a preset sampling frequency, performing signal data sampling processing on the real-time PPG signal data to generate a real-time PPG data sequence, and performing signal data sampling processing on the nominal PPG signal data to generate a nominal PPG data sequence, a data length L₁ of the real-time PPG data sequence is the same as a data length L₂ of the nominal PPG data sequence;
   according to a preset slot length, performing data slot division processing on the real-time PPG data sequence in sequence to obtain real-time PPG one-dimensional tensors, the number of which is the total number of real-time slots, and then fusing the real-time PPG one-dimensional tensors the number of which is the total number of real-time slots into one two-dimensional tensor to generate a real-time PPG two-dimensional tensor,
   wherein the total number of real-time slots= int(L₁/ slot length), int () is a rounding function, the shape of the real-time PPG one-dimensional tensor is 1×W₁, the W₁ is a one-dimensional parameter of the real-time PPG one-dimensional tensor, and W₁= slot length, the shape of the real-time PPG two-dimensional tensor is B₁×W₂, the B₁ is a two-dimensional parameter of the real-time PPG two-dimensional tensor, and B₁= the total number of real-time slots, the W₂ is a one-dimensional parameter of the real-time PPG two-dimensional tensor, and W₂=W₁;
   according to the slot length, performing data slot division processing on the nominal PPG data sequence in sequence to obtain nominal PPG one-dimensional tensors, the number of which is the total number of nominal slots, and then fusing the nominal PPG one-dimensional tensors the number of which is the total number of nominal slots into one two-dimensional tensor to generate a nominal PPG two-dimensional tensor,
   wherein the total number of nominal slots= int(L₂/ slot length), the shape of the nominal PPG one-dimensional tensor is 1×W₃, the W₃ is a one-dimensional parameter of the nominal PPG one-dimensional tensor, and W₃= slot length, the shape of the nominal PPG two-dimensional tensor is B₂×W₄, the B₂ is a two-dimensional parameter of the nominal PPG two-dimensional tensor, and B₂= the total number of nominal slots, the W₄ is a one-dimensional parameter of the nominal PPG two-dimensional tensor, and W₄=W₃;
   performing two-dimensional tensor fusion processing on the real-time PPG two-dimensional tensor and the nominal PPG two-dimensional tensor according to the sequence of nominal data following real-time data to generate a fused two-dimensional tensor,
   wherein the shape of the fused two-dimensional tensor is B₃×W₅, the B₃ is a two-dimensional parameter of the fused two-dimensional tensor, and B₃=B₂=B₁, the W₅ is a one-dimensional parameter of the fused two-dimensional tensor, and W₅=W₂+W₄, the fused two-dimensional tensor comprises the B₃ fused one-dimensional tensors, and the fused one-dimensional tensor is formed by splicing the corresponding real-time PPG one-dimensional tensor and the nominal PPG one-dimensional tensor according to the sequence of the nominal PPG one-dimensional tensor following the real-time PPG one-dimensional tensor; and
   according to a four-dimensional tensor input data format of the CNN model, raising the shape of the fused two-dimensional tensor from a two-dimensional tensor shape to a four-dimensional tensor shape to generate the CNN input four-dimensional tensor,
   wherein the shape of the CNN input four-dimensional tensor is B₄×H₁×W₆×C₁, the B₄ is a four-dimensional parameter of the CNN input four-dimensional tensor, and B₄=B₃, the H₁ is a three-dimensional parameter of the CNN input four-dimensional tensor, and H₁=2, the W₆ is a two-dimensional parameter of the CNN input four-dimensional tensor, and W₆=W₅/2, the C₁ is a one-dimensional parameter of the CNN input four-dimensional tensor, and C₁=1,
   wherein using the CNN model to perform multi-layer convolution pooling calculation on the CNN input four-dimensional tensor to generate a CNN output four-dimensional tensor specifically comprises:
      taking the CNN input four-dimensional tensor as a first input four-dimensional tensor, and then sending the first input four-dimensional tensor to a first convolutional network layer of the CNN model for first-layer convolution pooling calculation to generate a first output four-dimensional tensor; then, taking the first output four-dimensional tensor as a second input four-dimensional tensor, and sending the second input four-dimensional tensor to a second convolutional network layer of the CNN model for second-layer convolution pooling calculation to generate a second output four-dimensional tensor; and finally, taking a penultimate output four-dimensional tensor as a last input four-dimensional tensor, and sending the last input four-dimensional tensor to a last convolutional network layer of the CNN model for last-layer convolution pooling calculation to generate the CNN output four-dimensional tensor,
      wherein the CNN model comprises a plurality of convolutional network layers, the convolutional network layer comprises a convolution layer and a pooling layer, the shape of the CNN output four-dimensional tensor is B₅×H₂×W₇×C₂, the B₅ is a four-dimensional parameter of the CNN output four-dimensional tensor, and B₅=B₄, the H₂ is a three-dimensional parameter of the CNN output four-dimensional tensor, the W₇ is a two-dimensional parameter of the CNN output four-dimensional tensor, and the C₂ is a one-dimensional parameter of the CNN output four-dimensional tensor,
      wherein performing input data preparation processing on an ANN model according to the CNN output four-dimensional tensor to generate an ANN input two-dimensional tensor specifically comprises:
         according to a two-dimensional tensor input data format of the ANN model, reducing the shape of the CNN output four-dimensional tensor from a four-dimensional tensor shape to a two-dimensional tensor shape to generate the ANN input two-dimensional tensor,
         wherein the shape of the ANN input two-dimensional tensor is B₆×W'₁, the B₆ is a two-dimensional parameter of the ANN input two-dimensional tensor, and B₆=B₅, the W'₁ is a one-dimensional parameter of the ANN input two-dimensional tensor, and W'₁=H₂*W₇*C₂, the ANN input two-dimensional tensor comprises the B₆ ANN input one-dimensional tensors, the shape of the ANN input one-dimensional tensor is 1×W'₂, the W'₂ is a one-dimensional parameter of the ANN input one-dimensional tensor, and W'₂= W'₁; and
         adding the nominal diastolic blood pressure data and the nominal systolic blood pressure data to the end of each ANN input one-dimensional tensor, wherein the shape of the ANN input one-dimensional tensor becomes 1×W'₃, the W'₃ is a new one-dimensional parameter of the ANN input one-dimensional tensor, and W'₃=W'₂+2=W'₁+2=H₂*W₇*C₂+2, the shape of the ANN input two-dimensional tensor become B₆×W₈, the W₈ is a new one-dimensional parameter of the ANN input two-dimensional tensor, and W₈=W'₃=H₂*W₇*C₂+2,
         wherein using the ANN model to perform regression calculation on the ANN input two-dimensional tensor to generate an ANN output two-dimensional tensor specifically comprises:
            taking the ANN input two-dimensional tensor as a first input two-dimensional tensor, and then sending the first input two-dimensional tensor to a first fully connected layer of the ANN model for first-layer full connection calculation to generate a first output two-dimensional tensor; then, taking the first output two-dimensional tensor as a second input two-dimensional tensor, and sending the second input two-dimensional tensor to a second fully connected layer of the ANN model for second-layer full connection calculation to generate a second output two-dimensional tensor; and finally, taking a penultimate output two-dimensional tensor as a last input two-dimensional tensor, and sending the last input two-dimensional tensor to a last fully connected layer of the ANN model for last-layer full connection calculation to generate the ANN output two-dimensional tensor,
            wherein the ANN model comprises a plurality of fully connected layers, the shape of the ANN output two-dimensional tensor is B₇×W₉, the B₇ is a two-dimensional parameter of the ANN output two-dimensional tensor, and B₇=B₆, the W₉ is a one-dimensional parameter of the ANN output two-dimensional tensor, and W₉=2, the ANN output two-dimensional tensor comprises the B₇ ANN output one-dimensional tensors, and the ANN output one-dimensional tensor comprises relative diastolic blood pressure data and relative systolic blood pressure data,
            wherein performing blood pressure data calculation according to the nominal diastolic blood pressure data, the nominal systolic blood pressure data and the ANN output two-dimensional tensor to generate a blood pressure two-dimensional tensor specifically comprises:
               when the preset relative relation information is a difference relation, using the nominal diastolic blood pressure data to perform diastolic blood pressure value increase processing on all the relative diastolic blood pressure data in the ANN output two-dimensional tensor, using the nominal systolic blood pressure data to perform systolic blood pressure value increase processing on all the relative systolic blood pressure data in the ANN output two-dimensional tensor, and then, taking the ANN output two-dimensional tensor after the pressure value increasing process as the blood pressure two-dimensional tensor,
               wherein the shape of the blood pressure two-dimensional tensor is B₈xW₁₀, the B₈ is a two-dimensional parameter of the blood pressure two-dimensional tensor, and B₈=B₇, the W₁₀ is a one-dimensional parameter of the blood pressure two-dimensional tensor, and W₁₀=2, the blood pressure two-dimensional tensor comprises the B₈ blood pressure one-dimensional tensors, the blood pressure one-dimensional tensor comprises diastolic blood pressure data and systolic blood pressure data, the diastolic blood pressure data is the sum of the corresponding relative diastolic blood pressure data and the nominal diastolic blood pressure data, and the systolic blood pressure data is the sum of the corresponding relative systolic blood pressure data and the nominal systolic blood pressure data,
               wherein when preset prediction type information is a first type, performing mean blood pressure data calculation according to the blood pressure two-dimensional tensor to generate diastolic blood pressure prediction data and systolic blood pressure prediction data, and when prediction type information is a second type, performing blood pressure data extraction processing on the blood pressure two-dimensional tensor to generate a diastolic pressure prediction data sequence and a systolic pressure prediction data sequence specifically comprise:
                  when the prediction type information is the first type, calculating a mean value of all the diastolic blood pressure data in the blood pressure two-dimensional tensor to generate the diastolic blood pressure prediction data, and calculating a mean value of all the systolic blood pressure data in the blood pressure two-dimensional tensor to generate the systolic blood pressure prediction data; and
                  when the prediction type information is the second type, sequentially extracting the diastolic blood pressure data from the blood pressure two-dimensional tensor to form the diastolic blood pressure prediction data sequence, and extracting the systolic blood pressure data from the blood pressure two-dimensional tensor to form the systolic blood pressure prediction data sequence.

Further, sending the first input four-dimensional tensor to a first convolutional network layer of the CNN model for first-layer convolution pooling calculation to generate a first output four-dimensional tensor specifically comprises:
sending the first input four-dimensional tensor into a first convolution layer of the first convolutional network layer for first convolution calculation to generate a first convolution four-dimensional tensor, and sending the first convolution four-dimensional tensor into a first pooling layer of the first convolutional network layer for first pooling calculation to generate the first output four-dimensional tensor.

The second aspect of the embodiment of the invention provides electronic equipment, comprising a memory, a processor and a transceiver,
wherein the processor is configured to be coupled with the memory, and read and execute instructions in the memory, so as to implement the method steps in the first aspect,
the transceiver is coupled with the processor, and the processor controls the transceiver to send and receive messages.

The third aspect of the embodiment of the invention provides a computer program product, which comprises computer program code that, when executed by a computer, causes the computer to perform the method described in the first aspect.

The fourth aspect of the embodiment of the invention provides a computer-readable storage medium, which stores computer instructions that, when executed by a computer, cause the computer to execute the method described in the first aspect.

The embodiment of the invention provides a blood pressure prediction method and device fusing nominal PPG signal data, electronic equipment, a computer program product and a computer-readable storage medium. Firstly, the nominal PPG signal data and corresponding nominal blood pressure data (nominal diastolic blood pressure data and nominal systolic blood pressure data) of a test object are obtained, then a well-trained CNN+ANN artificial intelligence blood pressure prediction network for predicting relative blood pressure data is used to perform blood pressure prediction operation on the real-time PPG signal and nominal PPG signal of the test object to obtain relative blood pressure data (relative diastolic blood pressure data and relative systolic blood pressure data), and then absolute blood pressure data calculation is performed on the nominal blood pressure data and the relative blood pressure data according to relative relationship information to obtain the final blood pressure data. In this way, the prediction accuracy of an artificial intelligence blood pressure prediction network is improved.

### BRIEF DESCRIPTION OF THE DRAWINGS

Fig. 1 is a diagram of a blood pressure prediction method fusing nominal photoplethysmography (PPG) signal data provided in the first embodiment of the invention;
Fig. 2a is a structural schematic diagram of a convolutional neural network provided in the first embodiment of the invention;
Fig. 2b is a structural schematic diagram of an artificial neural network provided in the first embodiment of the invention;
Fig. 3 is a modular structural diagram of a blood pressure prediction device fusing nominal PPG signal data provided in the second embodiment of the invention; and
Fig. 4 is a structural schematic diagram of electronic equipment provided by the third embodiment of the invention.

### DETAILED DESCRIPTION OF THE INVENTION

In order to make the object, technical solution and advantages of the invention clearer, the invention will be described in further detail below with reference to the accompanying drawings. Obviously, the described embodiments are only a part of the embodiments of the present invention, but not all of them

Before the embodiment of the invention is described in detail, the structures and data formats of CNN and ANN in the artificial intelligence blood pressure prediction network mentioned above are briefly described.

A convolutional neural network (CNN), as we know, is one of the core networks in the field of feature recognition. When CNN is applied to the field of blood pressure feature recognition, feature extraction calculation is performed on one-dimensional PPG signal data, and after performing convolution operation and pooling operation on input original PPG signal data, reserves the feature data conforming to the characteristics of PPG signals for other networks to learn. The CNN model mentioned herein is a CNN model trained through feature extraction, and it is composed of multiple convolutional network layers, each of which is composed of a convolution layer and a pooling layer. The convolution layer is responsible for feature extraction calculation of the input data of the CNN model, the pooling layer performs down sampling operation to the extraction results of the convolution layer, and output data of each convolutional network layer will be used as input data of the next convolutional network layer. Here, the input data and output data of each convolutional network layer are in the form of four-dimensional tensors: the input four-dimensional tensor (in the shape of X₁₄×X₁₃×X₁₂×Xᵢ₁) and the output four-dimensional tensor (in the shape of Xₒ₄×Xₒ₃×Xₒ₂×Xₒ₁), wherein Xᵢ₄, Xᵢ₃, Xᵢ₂ and Xᵢ₁ are four, three, two and one-dimensional parameters of the input four-dimensional tensor respectively, and Xₒ₄, Xₒ₃, Xₒ₂ and Xₒ₁ are four, three, two and one-dimensional parameters of the output four-dimensional tensor respectively. After the calculation of each convolutional network layer, the shape of the output data relative to the shape of the input data, the variation rule of the dimensional parameters are as follows : (1) Xₒ₄ vs Xᵢ₄, the 4-dimensional parameter (corresponding to the total number of slots of the PPG signal data in this embodiment) does not change; (2) Xₒ₃ and Xₒ₂ vs Xᵢ₃ and Xᵢ₂, the three-dimensional and two-dimensional parameters change, and the change is related to the size of a convolution kernel and the setting of a sliding step of each convolution layer, as well as the size of a pooling window and a sliding step of the pooling layer; and (3) Xₒ₁ vs Xᵢ₁, the change of the one-dimensional parameter is related to a selected output space dimension (the number of convolution kernels) in the convolution layer.

An artificial neural network (ANN) refers to a complex network structure formed by a large number of interconnected neurons (nodes), which is some kind of abstraction, simplification and simulation of the organization structure and operation mechanism of the human brain. The ANN uses a mathematical model to simulate neuron activities, which is an information processing system based on imitating the structure and function of a brain neural network. The common application of the ANN is regression calculation of data. The ANN model mentioned in the application is an ANN model which has been trained by regression. Specifically, the ANN model is composed of multiple fully connected layers. Wherein, each fully connected layer includes a plurality of nodes, and each node is connected with all nodes of the previous layer so that summarize the node data extracted from the previous layer and perform the node calculation once, and take the calculation result as a value of the current node, and wait for the node of the next fully connected layer to connect and obtain it. The node calculation here is also called full connection calculation, comprising node connection calculation and activation calculation. Generally, the activation function uses the Rectified Linear Unit (ReLU) function in most cases, but other functions can be used to complete the calculation. Here, the input data and output data of each fully connected layer are in the form of two-dimensional tensors: input two-dimensional tensor (in the shape of Yᵢ₂×Yᵢ₁) and output two-dimensional tensor (in the shape of Yₒ₂×Yₒ₁), wherein Yᵢ₂ and Yᵢ₁ are the two-dimensional and one-dimensional parameters of the input two-dimensional tensor, and Yₒ₂ and Yₒ₁ are the two-dimensional and one-dimensional parameters of the output two-dimensional tensor. After the calculation of each fully connected layer, the shape of the output data relative to the shape of the input data, the variation rule of the dimensional parameters are as follows:: (1) Yₒ₂ vs Yᵢ₂, the 2-dimensional parameter (corresponding to the total number of slots of the PPG signal data in this embodiment) does not change; and (2) Yₒ₁ vs Yᵢ₁, the change of the one-dimensional parameter is related to the total number of nodes in the fully connected layer. Specifically, Yₒ₁ of the last fully connected layer in the embodiment of the invention is 2. According to the embodiment of the invention, the shape of a two-dimensional tensor finally output by the ANN model is the total number of slots ×2, and the two-dimensional tensor comprises one-dimensional tensors, the number of which is the total number of slots, and each one-dimensional tensor corresponds to one slot and comprises two blood pressure data related to the corresponding slots: relative diastolic blood pressure data and relative systolic blood pressure data.

In the embodiment of the present invention, because the output data of the CNN model need to be input into the ANN model, an output result of the CNN model needs to be reduced from the output four-dimensional tensor shape (Xₒ₄×Xₒ₃×Xₒ₂×Xₒ₁) to the input two-dimensional tensor shape (Yᵢ₂×Y'ᵢ₁) of the ANN model, wherein Y'ᵢ₁ is the one-dimensional parameter of the input two-dimensional tensor, Yᵢ₂= Xₒ₄, and Y'ᵢ₁= Xₒ₃*Xₒ₂*Xₒ₁. Here, the input two-dimensional tensor comprises Yᵢ₂ number of input one-dimensional tensors. In order to further improve the calculation accuracy of the ANN model, the embodiment of the present invention adds two data to an end of each input one-dimensional tensor: nominal diastolic blood pressure data and nominal systolic blood pressure data, thereby changing the shape of the input one-dimensional tensor from 1×Y'ᵢ₁ to 1×(Y'ᵢ₁+2), and accordingly, the shape of the input two-dimensional tensor is also changed from Yᵢ₂×Y'ᵢ₁ to Yᵢ₂×Yᵢ₁, wherein Yᵢ₁=Y'ᵢ₁+2=Xₒ₃*Xₒ₂*Xₒ₁+2.

Before the embodiment of the invention is described in detail, CNN and ANN application characteristics in the artificial intelligence blood pressure prediction network in the embodiment of the invention are briefly described.

A conventional artificial intelligence blood pressure prediction network directly segments real-time PPG signal data (real-time PPG signal data collected from a test object that need blood pressure prediction) (the reason why the PPG signal data are segmented into multiple PPG signal data slots is because an input length of the CNN model is limited), then uses the data as the input data of the CNN model, performs PPG signal feature extraction by means of the CNN model to obtain feature data, and then inputs the feature data into the ANN model, and performs regression calculation to obtain the absolute systolic blood pressure and diastolic blood pressure data corresponding to each PPG signal data slot. In practical application, we found that similar PPG signal data may be collected from different test subjects. If the above model is used, the absolute systolic blood pressure and diastolic blood pressure data of different test subjects calculated are almost the same. However, in reality, we found that due to individual differences (such as gender, age, height, weight, arm span, BMI, body temperature, whether the test object had coffee or strong tea before the test, or whether the test object exercised before the test), even if the collected PPG signal data are similar, blood pressure states may be different for different people.

Therefore, in order to improve the prediction accuracy of the artificial intelligence blood pressure prediction network, the embodiment of the present invention makes some adjustments to the CNN model and the ANN model of the artificial intelligence blood pressure prediction network respectively. The adjusted CNN model receives real-time PPG signal data as well as nominal PPG signal data at the same time, and the CNN model finally outputs relative feature data calculated according to a specific relative relationship between the real-time PPG signal data and the nominal PPG signal data, instead of absolute feature data of the real-time PPG signal data. Here, the specific relative relationship may be difference, ratio, logarithmic ratio, exponential ratio, etc. The input data of the adjusted ANN model changes from the absolute feature data to the relative feature data, and the output data changes from the absolute blood pressure data to the relative blood pressure data. Here, the so-called nominal PPG signal data are reference data which are specially collected for the test object and have the same time length as the real-time PPG signal data. While the nominal PPG signal data are collected, the nominal systolic blood pressure and the diastolic blood pressure data corresponding to the nominal PPG signal data are obtained through blood pressure measuring equipment. After relative systolic blood pressure data and diastolic blood pressure data are obtained from the output data of the ANN model, the absolute blood pressure can be reversely deduced according to the specific relative relationship (such as difference, ratio, logarithmic ratio, exponential ratio, etc.) combined with the nominal diastolic blood pressure data and nominal systolic blood pressure data, and finally predicted systolic blood pressure and diastolic blood pressure data corresponding to the real-time PPG signal data can be obtained.

By using the artificial intelligence blood pressure prediction network (CNN+ANN) which fuses the nominal PPG signal data with the real-time PPG signal data, when predicting the real-time PPG signal data collected from different objects, even if the real-time data are similar, the obvious relative differences can be obtained because of the differences between the nominal PPG signal data, so that the differences between the finally predicted blood pressure data will be obvious. Therefore, the problem of measurement inaccuracy of the conventional prediction network is solved, and the prediction accuracy of the artificial intelligence blood pressure prediction network is further improved.

The first embodiment of the present invention provides a blood pressure prediction method fusing nominal PPG signal data, which uses an artificial intelligence blood pressure prediction network (CNN+ANN) for predicting relative blood pressure data to perform prediction operations on input data which fused nominal PPG signal data with real-time PPG signal data to obtain relative blood pressure data, and then performs inverse deduction for absolute blood pressure data according to the specific relative relationship between nominal blood pressure data and relative blood pressure data to obtain the predicted blood pressure data corresponding to a real-time PPG signal. By the method provided in the first embodiment of the present invention, the prediction accuracy of the artificial intelligence blood pressure prediction network can be improved.

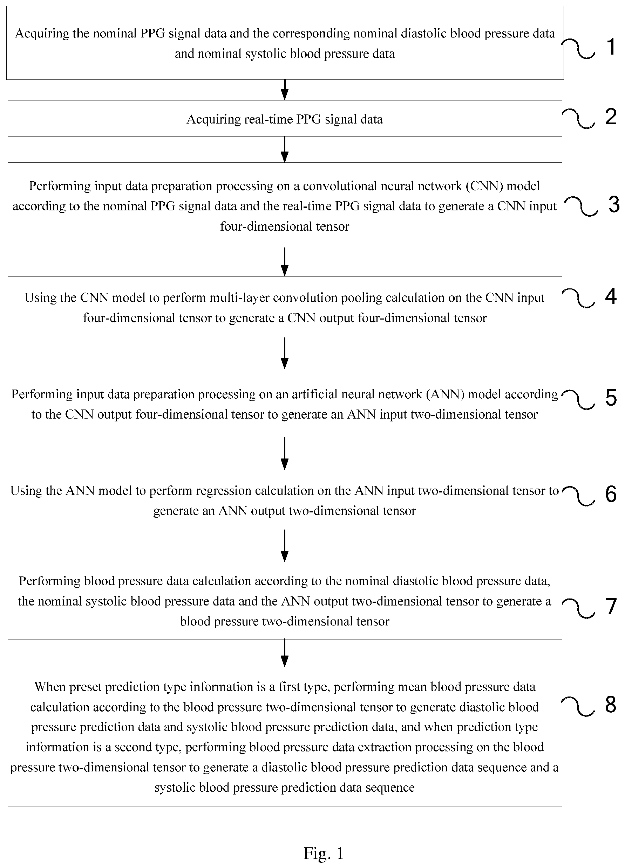

Fig. 1 is a diagram of a blood pressure prediction method fusing nominal PPG signal data provided in the first embodiment of the invention, As shown in the Fig. 1, the method mainly comprises the following steps.

Step 1, acquiring the nominal PPG signal data and the corresponding nominal diastolic blood pressure data and nominal systolic blood pressure data.

Specifically, equipment can acquire the nominal PPG signal data and the nominal diastolic blood pressure data and nominal systolic blood pressure data corresponding to the nominal PPG signal data from a local storage medium, and the equipment can also acquire the nominal PPG signal data, the nominal diastolic blood pressure data and the nominal systolic blood pressure data from storage media of other terminal equipment, servers or databases connected to the equipment.

Here, the equipment is specifically a terminal equipment or a server for implementing the method provided by the embodiment of the invention, the nominal PPG signal data is a piece of PPG signal data collected in advance for a test object, the corresponding nominal diastolic blood pressure data and nominal systolic blood pressure data are the actual blood pressure data obtained by measuring the blood pressure of the test object with blood pressure measuring equipment while the nominal PPG signal data are collected.

For example, the acquired nominal PPG signal data has a length of 10 seconds, corresponding to the nominal diastolic blood pressure data of 74 mmHg and the nominal systolic blood pressure data of 113 mmHg.

Step 2, acquiring real-time PPG signal data,
wherein a time length of the real-time PPG signal data is the same as that of the nominal PPG signal data.

Specifically, the equipment can perform real-time PPG signal collection and data sampling on the test object with its own PPG signal collection device, so as to obtain the real-time PPG signal data, and the equipment can also perform real-time PPG signal collection and data sampling on the test object through a PPG signal collection device connected to itself, so as to obtain the real-time PPG signal data.

For example, the acquired real-time PPG signal data has a length of 10 seconds, which is the same as the length of the nominal PPG signal data.

Step 3, performing input data preparation processing on a CNN model according to the nominal PPG signal data and the real-time PPG signal data to generate a CNN input four-dimensional tensor.

Specifically comprises the following steps: Step 31, according to a preset sampling frequency, performing signal data sampling processing on the real-time PPG signal data to generate a real-time PPG data sequence, and performing signal data sampling processing on the nominal PPG signal data to generate a nominal PPG data sequence.

Wherein the data length L₁ of the real-time PPG data sequence is the same as the data length L₂ of the nominal PPG data sequence.

Here, the sampling frequency is stored in the local storage medium of the equipment.

For example, if the real-time and nominal PPG signal data both have a length of 10 seconds, and a sampling frequency threshold is 125 Hz, then data lengths of the real-time and nominal PPG data sequences are L₁=L₂=10*125=1250, the real-time PPG data sequence is specifically a real-time PPG data sequence [1250], and the nominal PPG data sequence is specifically a nominal PPG data sequence [1250].

Step 32, according to a preset slot length, performing data slot division processing on the real-time PPG data sequence in sequence to obtain real-time PPG one-dimensional tensors, the number of which is the total number of real-time slots, and then fusing the real-time PPG one-dimensional tensors the number of which is the total number of real-time slots into one two-dimensional tensor to generate a real-time PPG two-dimensional tensor.

Wherein the total number of real-time slots= int(L₁/ slot length), int () is a rounding function, the shape of the real-time PPG one-dimensional tensor is 1×W₁, the W₁ is a one-dimensional parameter of the real-time PPG one-dimensional tensor, and W₁= slot length, the shape of the real-time PPG two-dimensional tensor is B₁×W₂, the B₁ is a two-dimensional parameter of the real-time PPG two-dimensional tensor, and B₁= the total number of real-time slots, the W₂ is a one-dimensional parameter of the real-time PPG two-dimensional tensor, and W₂=W₁.

Here, the slot length is stored in the local storage medium of the equipment.

Here, the value of the slot length is determined by the length of the input data of the CNN model, and slot division is performed by a sequential slot division method, so the data between adjacent slots do not coincide.

For example, if the slot length is 250, data slot division processing is performed on the real-time PPG data sequence [1250] in sequence, then B₁= the total number of real-time slots=int(1250/250)=5, W₂=W₁=250, and the shape of the real-time PPG two-dimensional tensor is specifically 5×250, which is expressed here as the real-time PPG two-dimensional tensor [5,250].

Step 33, according to the slot length, performing data slot division processing on the nominal PPG data sequence in sequence to obtain nominal PPG one-dimensional tensors, the number of which is the total number of nominal slots, and then fusing the nominal PPG one-dimensional tensors into one two-dimensional tensor to generate a nominal PPG two-dimensional tensor, wherein the number of the nominal PPG one-dimensional tensors is the total number of nominal slots.

Wherein the total number of nominal slots= int(L₂/ slot length), the shape of the nominal PPG one-dimensional tensor is 1×W₃, the W₃ is a one-dimensional parameter of the nominal PPG one-dimensional tensor, and W₃= slot length, the shape of the nominal PPG two-dimensional tensor is B₂×W₄, the B₂ is a two-dimensional parameter of the nominal PPG two-dimensional tensor, and B₂= the total number of nominal slots, the W₄ is a one-dimensional parameter of the nominal PPG two-dimensional tensor, and W₄=W₃.

For example, if the slot length is 250, data slot division processing is performed on the nominal PPG data sequence [1250] in sequence, then B₂= the total number of nominal slots=int(12501250)=5, W₄=W₃=250, and the shape of the nominal PPG two-dimensional tensor is specifically 5×250, which is expressed here as the nominal PPG two-dimensional tensor [5,250].

Step 34, performing two-dimensional tensor fusion processing on the real-time PPG two-dimensional tensor and the nominal PPG two-dimensional tensor according to the sequence of nominal data following real-time data to generate a fused two-dimensional tensor.

Wherein the shape of the fused two-dimensional tensor is B₃×W₅, the B₃ is a two-dimensional parameter of the fused two-dimensional tensor, and B₃=B₂=B₁, the W₅ is a one-dimensional parameter of the fused two-dimensional tensor, and W₅=W₂+W₄, the fused two-dimensional tensor comprises the B₃ fused one-dimensional tensors, and the fused one-dimensional tensor is formed by splicing the corresponding real-time PPG one-dimensional tensor and the nominal PPG one-dimensional tensor according to the sequence of the nominal PPG one-dimensional tensor following the real-time PPG one-dimensional tensor.

For example, the data of the real-time PPG two-dimensional tensor [5,250] are specifically {(Z_{1,1},...Z_{1,250}),(Z_{2,1},...Z_{2,250}),(Z_{3,1},...Z_{3,250}),(Z_{4,1},...Z_{4,250}),(Z_{5,1},...Z_{5,250})}, wherein (Z_{1,1},...Z_{1,250}), (Z_{2,1},...Z_{2,250}), (Z_{3,1},...Z_{3,250}), (Z_{4,1},...Z_{4,250}), (Z_{5,1},...Z_{5,250}) are the data of the first, second, third, fourth and fifth real-time PPG one-dimensional tensors in the real-time PPG two-dimensional tensor respectively.

The data of the nominal PPG two-dimensional tensor [5,250] are specifically {(D_{1,1},...D_{1,250}),(D_{2,1},...D_{2,250}),(D_{3,1},...D_{3,250}),(D_{4,1},...D_{4,250}),(D_{5,1},...D_{5,250})}, wherein (D_{1,1},...D_{1,250}), (D_{2,1},...D_{2,250}), (D_{3,1},...D_{3,250}), (D_{4,1},...D_{4,250}), (D_{5,1},...D_{5,250}) are the data of the first, second, third, fourth and fifth nominal PPG one-dimensional tensors in the nominal PPG two-dimensional tensor respectively.

Then, after two-dimensional tensor fusion processing of the real-time PPG two-dimensional tensor [5,250] and the nominal PPG two-dimensional tensor [5,250], B₃=B₂=B₁=5, W₅=W₂+W₄=250+250=500, and the shape of the fused two-dimensional tensor is 5×500, which is expressed here as the fused two-dimensional tensor [5, 500].

The fused two-dimensional tensor [5,500] comprises five fused one-dimensional tensors, and each fused one-dimensional tensor comprises W₅=W₂+W₄=250+250=500 data, among which the first 250 data are the data of the corresponding real-time PPG one-dimensional tensors in the real-time PPG two-dimensional tensor [5,250], and the last 250 data are the data of the corresponding nominal PPG one-dimensional tensors in the nominal PPG two-dimensional tensor [5,250].

That is, the data format of the fused two-dimensional tensor should be:
{(Z_{1,1},...Z_{1,250},D_{1,1},...D_{1,250}),(Z_{2,1},...Z_{2,250},D_{2,1},...D_{2,250}),(Z_{3,1},...Z_{3,250},D_{3,1}, ...D_{3,250}),(Z_{4,1},...Z_{4,250},D_{4,1},...D_{4,250}),(Z_{5,1},...Z_{5,250},D_{5,1},...D_{5,250})

Wherein,
(Z_{1,1},...Z_{1,250},D_{1,1},...D_{1,250}),(Z_{2,1},...Z_{2,250},D_{2,1},...D_{2,250}),(Z_{3,1},...Z_{3,250},D_{3,1},... D_{3,250}),(Z_{4,1},...Z_{4,250},D_{4,1},...D_{4,250}),(Z_{5,1},...Z_{5,250},D_{5,1},...D_{5,250}) are the data of the first, second, third, fourth and fifth fused one-dimensional tensors in the fused two-dimensional tensor respectively.

Step 35, according to a four-dimensional tensor input data format of the CNN model, raising the shape of the fused two-dimensional tensor from a two-dimensional tensor shape to a four-dimensional tensor shape to generate the CNN input four-dimensional tensor.

Wherein the shape of the CNN input four-dimensional tensor is B₄×H₁×W₆×C₁, the B₄ is a four-dimensional parameter of the CNN input four-dimensional tensor, and B₄=B₃, the H₁ is a three-dimensional parameter of the CNN input four-dimensional tensor, and H₁=2, the W₆ is a two-dimensional parameter of the CNN input four-dimensional tensor, and W₆=W₅/2, the C₁ is a one-dimensional parameter of the CNN input four-dimensional tensor, and C₁=1.

Here, the shape of the fused two-dimensional tensor is raised from the two-dimensional tensor shape to the four-dimensional tensor shape, and the process only resets the tensor shape without destroying the actual data order in the tensor.

For example, the fused two-dimensional tensor [5,500] is raised from the two-dimensional tensor shape to the four-dimensional tensor shape, B₄=B₃=5, W₆=W₅/2=5/2=250, and the shape of the obtained CNN input four-dimensional tensor is 5×2×250×1, which is represented here as the CNN input four-dimensional tensor [5,2,250,1].

Step 4, using the CNN model to perform multi-layer convolution pooling calculation on the CNN input four-dimensional tensor to generate a CNN output four-dimensional tensor.

Which specifically comprises: taking the CNN input four-dimensional tensor as a first input four-dimensional tensor, and then sending the first input four-dimensional tensor to a first convolutional network layer of the CNN model for first-layer convolution pooling calculation to generate a first output four-dimensional tensor. Then, taking the first output four-dimensional tensor as a second input four-dimensional tensor, and sending the second input four-dimensional tensor to a second convolutional network layer of the CNN model for second-layer convolution pooling calculation to generate a second output four-dimensional tensor. And finally, taking a penultimate output four-dimensional tensor as a last input four-dimensional tensor, and sending the last input four-dimensional tensor to a last convolutional network layer of the CNN model for last-layer convolution pooling calculation to generate the CNN output four-dimensional tensor.

Wherein the CNN model comprises a plurality of convolutional network layers, the convolutional network layer comprises a convolution layer and a pooling layer, the shape of the CNN output four-dimensional tensor is B₅×H₂×W₇×C₂, the B₅ is a four-dimensional parameter of the CNN output four-dimensional tensor, and B₅=B₄, the H₂ is a three-dimensional parameter of the CNN output four-dimensional tensor, the W₇ is a two-dimensional parameter of the CNN output four-dimensional tensor, and the C₂ is a one-dimensional parameter of the CNN output four-dimensional tensor.

Wherein, sending the first input four-dimensional tensor to a first convolutional network layer of the CNN model for first-layer convolution pooling calculation to generate a first output four-dimensional tensor specifically comprises sending the first input four-dimensional tensor into a first convolution layer of the first convolutional network layer for first convolution calculation to generate a first convolution four-dimensional tensor, and sending the first convolution four-dimensional tensor into a first pooling layer of the first convolutional network layer for first pooling calculation to generate the first output four-dimensional tensor.

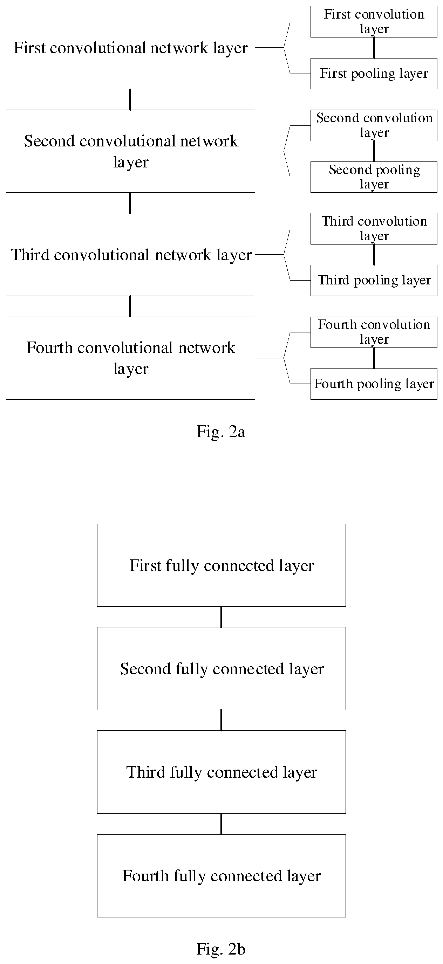

For example,Fig. 2a is a structural diagram of a CNN provided in the first embodiment of the invention, as shown in the Fig.2a, the total number of layers of the CNN model is four, then the following steps are performed.

Taking the CNN input four-dimensional tensor as the first input four-dimensional tensor, sending the first input four-dimensional tensor into a first convolution layer of the first convolutional network layer of the CNN model for first convolution calculation to generate a first convolution four-dimensional tensor, and sending the first convolution four-dimensional tensor into a first pooling layer of the first convolutional network layer for first pooling calculation to generate the first output four-dimensional tensor.

Taking the first output four-dimensional tensor as the second input four-dimensional tensor, sending the second input four-dimensional tensor into a second convolution layer of the second convolutional network layer of the CNN model for second convolution calculation to generate a second convolution four-dimensional tensor, and sending the second convolution four-dimensional tensor into a second pooling layer of the second convolutional network layer for second pooling calculation to generate the second output four-dimensional tensor.

Taking the second output four-dimensional tensor as a third input four-dimensional tensor, sending the third input four-dimensional tensor into a third convolution layer of a third convolutional network layer of the CNN model for third convolution calculation to generate a third convolution four-dimensional tensor, and sending the third convolution four-dimensional tensor into a third pooling layer of the third convolutional network layer for third pooling calculation to generate a third output four-dimensional tensor; and

Taking the third output four-dimensional tensor as the fourth input four-dimensional tensor, sending the fourth input four-dimensional tensor into a fourth convolution layer of a fourth convolutional network layer of the CNN model for fourth convolution calculation to generate a fourth convolution four-dimensional tensor, and sending the fourth convolution four-dimensional tensor into a fourth pooling layer of the fourth convolutional network layer for fourth pooling calculation to generate a fourth output four-dimensional tensor; here, the fourth output four-dimensional tensor is the finally output CNN output four-dimensional tensor.

As can be seen from the above, in the CNN model, after each convolution layer or pooling layer, the shape of the input data will change, but the four-dimensional tensor form does not change, and the four-dimensional parameter (total number of slots) will not change. The change of the three-dimensional and second-dimensional parameters is related to the size of a convolution kernel and the setting of a sliding step of each convolution layer, as well as the size of a pooling window and a sliding step of the pooling layer, and the change of the one-dimensional parameter is related to a selected output space dimension (the number of convolution kernels) in the convolution layer. In practical application, the setting of the number of layers in the network and various parameters of each layer should be constantly revised according to experience and experimental results.

Step 5, performing input data preparation processing on an artificial neural network (ANN) model according to the CNN output four-dimensional tensor to generate an ANN input two-dimensional tensor.

Which specifically comprises the following steps: Step 51, according to a two-dimensional tensor input data format of the ANN model, reducing the shape of the CNN output four-dimensional tensor from a four-dimensional tensor shape to a two-dimensional tensor shape to generate the ANN input two-dimensional tensor, wherein the shape of the ANN input two-dimensional tensor is B₆xW'₁, the B₆ is a two-dimensional parameter of the ANN input two-dimensional tensor, and B₆=B₅, the W'₁ is a one-dimensional parameter of the ANN input two-dimensional tensor, and W'₁=H₂*W₇*C₂, the ANN input two-dimensional tensor comprises the B₆ ANN input one-dimensional tensors, the shape of the ANN input one-dimensional tensor is 1×W'₂, the W'₂ is a one-dimensional parameter of the ANN input one-dimensional tensor, and W'₂= W'₁.

Here, reducing the shape of the CNN output four-dimensional tensor from the four-dimensional tensor shape to the second-dimensional tensor shape is actually reducing the CNN output four-dimensional tensor from four-dimensional to two-dimensional, and the process only resets the tensor shape without destroying the actual data order in the tensor.

For example, if the shape of the CNN output four-dimensional tensor is 5×2×20×64, then B₆=B₅=5, W₈=H₂*W₇*C₂=2*20*64=2560, and the shape of the ANN input two-dimensional tensor should be 5×2560, which is represented here as the ANN input two-dimensional tensor [5,2560]. Correspondingly, the ANN input two-dimensional tensor comprises five ANN input one-dimensional tensors, and the shape of the ANN input one-dimensional tensor should be 1×2560, which is represented here as the ANN input one-dimensional tensor [2560].

Step 52, adding the nominal diastolic blood pressure data and the nominal systolic blood pressure data to an end of each ANN input one-dimensional tensor, wherein the shape of the ANN input one-dimensional tensor becomes 1×W'₃, the W'₃ is a one-dimensional parameter of the ANN input one-dimensional tensor, and W'₃=W'₂+2=W'₁+2=H₂*W₇*C₂+2, the shape of the ANN input two-dimensional tensor become B₆×W₈, the W₈ is a new one-dimensional parameter of the ANN input two-dimensional tensor, and W₈=W'₃=H₂*W₇*C₂+2.

Here, two data, that is, the nominal diastolic blood pressure data and the nominal systolic blood pressure data are added to an end of each ANN input one-dimensional tensor, so as to improve the calculation accuracy of the ANN model, and after data addition, the shape of the ANN input one-dimensional tensor is changed from 1×W'₂ to 1×W'₃, and accordingly, the shape of the ANN input two-dimensional tensor is changed from B₆×W'₁ to B₆×W₈.

For example, the shape of the ANN input two-dimensional tensor before data addition is 5×2560, and the shape of the ANN input one-dimensional tensor before data addition is 1× 2560, and after data addition, the shape of the ANN input one-dimensional tensor becomes 1×2562, and the shape of the ANN input two-dimensional tensor becomes 5×2562, which is represented here as the ANN input two-dimensional tensor [5,2562].

Step 6, using the ANN model to perform regression calculation on the ANN input two-dimensional tensor to generate an ANN output two-dimensional tensor.

Specifically comprising: taking the ANN input two-dimensional tensor as a first input two-dimensional tensor, and then sending the first input two-dimensional tensor to a first fully connected layer of the ANN model for first-layer full connection calculation to generate a first output two-dimensional tensor; then, taking the first output two-dimensional tensor as a second input two-dimensional tensor, and sending the second input two-dimensional tensor to a second fully connected layer of the ANN model for second-layer full connection calculation to generate a second output two-dimensional tensor; and finally, taking a penultimate output two-dimensional tensor as a last input two-dimensional tensor, and sending the last input two-dimensional tensor to a last fully connected layer of the ANN model for last-layer full connection calculation to generate the ANN output two-dimensional tensor.

Wherein the ANN model comprises a plurality of fully connected layers, the shape of the ANN output two-dimensional tensor is B₇×W₉, the B₇ is a two-dimensional parameter of the ANN output two-dimensional tensor, and B₇=B₆, the W₉ is a one-dimensional parameter of the ANN output two-dimensional tensor, and W₉=2, the ANN output two-dimensional tensor comprises the B₇ ANN output one-dimensional tensors, and the ANN output one-dimensional tensor comprises relative diastolic blood pressure data and relative systolic blood pressure data.

For example, Fig. 2b which is a structural diagram of an ANN provided in the first embodiment of the invention, as shown in Fig.2b, the ANN model comprises four fully connected layers, then the following steps are performed:

Taking the ANN input two-dimensional tensor as a first input two-dimensional tensor, and then sending the first input two-dimensional tensor to a first fully connected layer of the ANN model for first-layer full connection calculation to generate a first output two-dimensional tensor.

Then, taking the first output two-dimensional tensor as a second input two-dimensional tensor, and sending the second input two-dimensional tensor to a second fully connected layer of the ANN model for second-layer full connection calculation to generate a second output two-dimensional tensor.

Then, taking the second output two-dimensional tensor as a third input two-dimensional tensor, and sending the third input two-dimensional tensor to a third fully connected layer of the ANN model for third-layer full connection calculation to generate a third output two-dimensional tensor. And

Finally, taking the third output two-dimensional tensor as a fourth input two-dimensional tensor, and sending the fourth input two-dimensional tensor to a fourth fully connected layer of the ANN model for fourth-layer full connection calculation to generate a fourth output two-dimensional tensor; here, the fourth output two-dimensional tensor is the finally output ANN output two-dimensional tensor.

As can be seen from the above, the ANN model is composed of fully connected layers, and each node of the fully connected layer is connected with all nodes of the previous layer so that to summarize the features extracted from the previous layer. The number of nodes and activation function (ReLU is more common, but others can be used too) of each fully connected layer can be set. In this embodiment, the number of nodes in the last fully connected layer of the ANN model is 2, and the shape of the final corresponding output two-dimensional tensor is B₇×2.

For example, the ANN model comprises four fully connected layers, the shape of the ANN input two-dimensional tensor is 5×2560, then B₇=B₆=5, and the shape of the output two-dimensional tensor is 5×2, which is expressed here as the output two-dimensional tensor [5,2], and
the data in the ANN output two-dimensional tensor [5,2] should be
{(R_{db1},R_{sb1}),(R_{db2},R_{sb2}),(R_{db3},R_{sb3}),(R_{db4},R_{sb4}),(R_{db5},R_{sb5})},
wherein (R_{db1},R_{sb1}),(R_{db2},R_{sb2}),(R_{db3},R_{sb3}),(R_{db4},R_{sb4}),(R_{db5},R_{sb5}) are the data of first, second, third, fourth and fifth ANN output one-dimensional tensors in the ANN output two-dimensional tensor respectively (R_{sbi} is relative systolic blood pressure data, R_{dbi} is relative diastolic blood pressure data, and the value of i is from 1 to 5).

Step 7, performing blood pressure data calculation according to the nominal diastolic blood pressure data, the nominal systolic blood pressure data and the ANN output two-dimensional tensor to generate a blood pressure two-dimensional tensor.

Specifically comprising: when the preset relative relation information is a difference relation, using the nominal diastolic blood pressure data to perform diastolic blood pressure value increase processing on all the relative diastolic blood pressure data in the ANN output two-dimensional tensor, using the nominal systolic blood pressure data to perform systolic blood pressure value increase processing on all the relative systolic blood pressure data in the ANN output two-dimensional tensor, and then, taking the ANN output two-dimensional tensor after the pressure value increasing process as the blood pressure two-dimensional tensor.

Wherein the shape of the blood pressure two-dimensional tensor is B₈×W₁₀, the B₈ is a two-dimensional parameter of the blood pressure two-dimensional tensor, and B₈=B₇, the W₁₀ is a one-dimensional parameter of the blood pressure two-dimensional tensor, and W₁₀=2, the blood pressure two-dimensional tensor comprises the B₈ blood pressure one-dimensional tensors, the blood pressure one-dimensional tensor comprises diastolic blood pressure data and systolic blood pressure data, the diastolic blood pressure data is the sum of the corresponding relative diastolic blood pressure data and the nominal diastolic blood pressure data, and the systolic blood pressure data is the sum of the corresponding relative systolic blood pressure data and the nominal systolic blood pressure data.

Here, the relative relationship information comprises at least the difference relative relationship (difference relationship).

Here, in the first embodiment, when the relative relationship information is specifically the difference relationship, it means that the diastolic blood pressure data can be obtained by adding the relative diastolic blood pressure data to the nominal diastolic blood pressure data in the ANN output two-dimensional tensor, and the systolic blood pressure data can be obtained by adding the relative systolic blood pressure data to the nominal systolic blood pressure data.

For example,
the ANN output two-dimensional tensor [5,2] is {(7,23), (6,22), (7,21), (11,20), (9,18)},
the nominal diastolic blood pressure data are 74 mmHg, the nominal systolic blood pressure data are 113 mmHg, B₈=B₇=5, W₁₀=2, and the shape of the blood pressure two-dimensional tensor is 5×2 (here it is expressed as the blood pressure two-dimensional tensor [5,2]).

Then, the data of the blood pressure two-dimensional tensor [5,2] should be:
{(7+74,23+113),(6+74,22+113),(7+74,21+113),(11+74,20+113),(9+74,18+ 113)}={(81,136),(80,135),(81,134),(85,133),(83,131)}.

Step 8, when preset prediction type information is a first type, performing mean blood pressure data calculation according to the blood pressure two-dimensional tensor to generate diastolic blood pressure prediction data and systolic blood pressure prediction data, and when prediction type information is a second type, performing blood pressure data extraction processing on the blood pressure two-dimensional tensor to generate a diastolic pressure prediction data sequence and a systolic pressure prediction data sequence.

Specifically comprising: when the prediction type information is the first type, calculating a mean value of all the diastolic blood pressure data in the blood pressure two-dimensional tensor to generate the diastolic blood pressure prediction data, and calculating a mean value of all the systolic blood pressure data in the blood pressure two-dimensional tensor to generate the systolic blood pressure prediction data; and when the prediction type information is the second type, sequentially extracting the diastolic blood pressure data from the blood pressure two-dimensional tensor to form the diastolic blood pressure prediction data sequence, and extracting the systolic blood pressure data from the blood pressure two-dimensional tensor to form the systolic blood pressure prediction data sequence.

Here, the embodiment of the invention supports two types of prediction data output: the first type is to output a pair of mean blood pressure prediction data (diastolic blood pressure prediction data and systolic blood pressure prediction data), and the second type is to extract blood pressure data by slots to form dynamic blood pressure data sequences (diastolic blood pressure prediction data sequence and systolic blood pressure prediction data sequence). The prediction type information is stored in the local storage medium of the equipment, and the embodiment of the invention decides which type to use to output the prediction data by reading the content of the prediction type information.

For example, if the prediction type information is the first type and the data of the blood pressure two-dimensional tensor [5,2] are {(81,136), (80,135), (81,134), (85,133), (83,131)}, then:
The diastolic blood pressure prediction data=(81+80+81+85+83)/5=82(mmHg), and
The systolic blood pressure prediction data= (136 + 135 + 134 + 133 + 131) /5≈134(mmHg).

For another example, if the prediction type information is the second type and the data of the blood pressure two-dimensional tensor [5,2] are {(81,136),(80,135),(81,134),(85,133),(83,131)}, then:
The data content of the diastolic blood pressure prediction data sequence [5] is specifically (81,80,81,85,83), and
the data content of the systolic blood pressure prediction data sequence [5] is specifically (136,135,134,133,131).

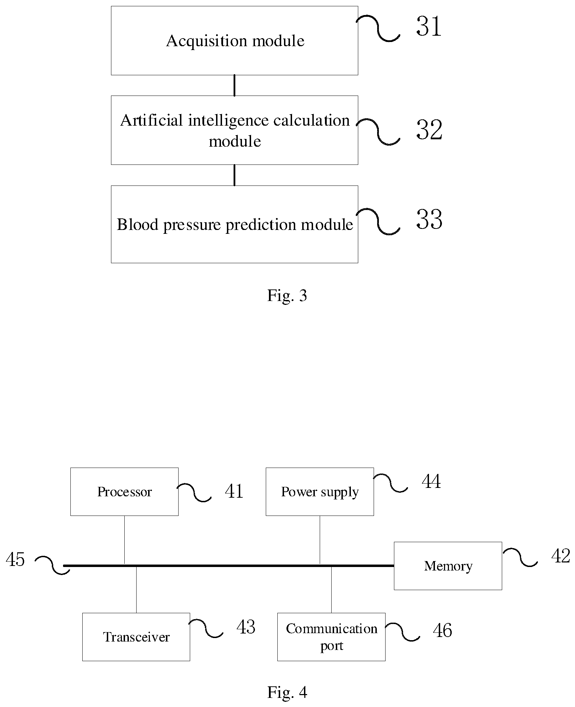

Fig. 3 is a modular structural diagram of a blood pressure prediction device fusing nominal PPG signal data provided in the second embodiment of the invention. The device may be the terminal equipment or server described in the previous embodiment, or may be a device which enables the terminal equipment or server to implement the method provided in the embodiment of the present invention, for example, the device may be a device or chip system of the above-mentioned terminal equipment or server. As shown in Fig. 3, the device comprises:
an acquisition module 31 for acquiring nominal PPG signal data and corresponding nominal diastolic blood pressure data and nominal systolic blood pressure data, and acquiring real-time PPG signal data, a time length of the real-time PPG signal data being the same as that of the nominal PPG signal data.
an artificial intelligence calculation module 32 for performing input data preparation processing on a CNN model according to the nominal PPG signal data and the real-time PPG signal data to generate a CNN input four-dimensional tensor, using the CNN model to perform multi-layer convolution pooling calculation on the CNN input four-dimensional tensor to generate a CNN output four-dimensional tensor, performing input data preparation on an ANN model according to the CNN output four-dimensional tensor to generate an ANN input two-dimensional tensor, and using the ANN model to perform regression calculation on the ANN input two-dimensional tensor to generate an ANN output two-dimensional tensor; and
a blood pressure prediction module 33 for performing blood pressure data calculation according to the nominal diastolic blood pressure data, the nominal systolic blood pressure data and the ANN output two-dimensional tensor to generate a blood pressure two-dimensional tensor, performing mean blood pressure data calculation according to the blood pressure two-dimensional tensor to generate diastolic blood pressure prediction data and systolic blood pressure prediction data when preset prediction type information is a first type, and performing blood pressure data extraction processing on the blood pressure two-dimensional tensor to generate a diastolic blood pressure prediction data sequence and a systolic blood pressure prediction data sequence when prediction type information is a second type.

The blood pressure prediction device fusing nominal PPG signal data provided by the embodiment of the present invention can implement the method steps in the above method embodiment, and the implementation principles and technical effects are similar, which will not be repeated here.

It should be understood that the division of different modules of the above device is based on logical functions, and in actual implementation, all or part of the modules can be integrated into a physical entity, or they can be physically separated. These modules can all be implemented in the form of software which can be called by processing elements, or all of them can be implemented in the form of hardware, or some modules are implemented in the form of software which can be called by processing elements, and some modules are implemented in the form of hardware. For example, the acquisition module may be a separate processing element, or may be integrated into a certain chip of the above-mentioned device, or it may be stored in a memory of the above-mentioned device in the form of program code, and called by a certain processing element of the above-mentioned device to implement the functions of the above-mentioned determination module. Other modules are implemented similarly. In addition, all or part of these modules may be integrated or implemented separately. The processing element described here may be an integrated circuit with signal processing capability. In the implementation process, each step of the above method or each module may be realized by an integrated logic circuit of hardware in the processor element or instructions in the form of software.

For example, the above modules may be one or more integrated circuits configured to implement the above method, such as one or more application specific integrated circuits (ASIC), one or more digital signal processors (DSP), one or more field programmable gate arrays (FPGA), etc. For another example, when one of the above modules is implemented in the form of a program code which can be called by a processing element, the processing element may be a general purpose processor, such as a central processing unit (CPU) or other processors which can call the program code. For example, these modules can be integrated and implemented in the form of system-on-a-chip (SOC).

In the above embodiments, the functional units can be implemented in whole or in part by software, hardware, firmware or any combination thereof. When implemented by software, the functional units can be implemented in whole or in part by computer program products. The computer program product comprises one or more computer instructions. When the computer program instructions are loaded and executed on a computer, the flow or function according to the embodiment of the invention is generated in whole or in part. The computer may be a general purpose computer, a special purpose computer, a computer network, or other programmable devices. The computer instructions may be stored in the computer-readable storage medium or transmitted from one computer-readable storage medium to another. For example, the computer instructions may be transmitted from one website, computer, server or data center to another website, computer, server or data center through wired (such as coaxial cable, optical fiber, digital subscriber line (DSL)) or wireless (such as infrared, wireless, Bluetooth and microwave) methods. The computer readable storage medium may be any available medium that can be accessed by a computer, or a data storage device such as server and data center that contains one or more available media integrations. The available medium may be magnetic medium (e.g., floppy disk, hard disk, magnetic tape), optical medium (e.g., DVD), or semiconductor medium (e.g. solid state disk (SSD)).

Fig. 4 is a structural schematic diagram of electronic equipment provided by the third embodiment of the invention. The electronic equipment may be the aforementioned terminal equipment or server, or may be terminal equipment or server connected to the aforementioned terminal equipment or server to realize the method of the embodiment of the present invention. As shown in Fig. 4, the electronic equipment may comprise a processor 41 (for example, CPU), a memory 42, and a transceiver 43. The transceiver 43 is coupled to the processor 41, and the processor 41 controls the transceiving action of the transceiver 43. The memory 42 may store various instructions for accomplishing various processing functions and realizing the method and processing procedures provided in the above embodiments of the invention. Preferably, the electronic equipment according to the embodiment of the invention further comprises a power supply 44, a system bus 45 and a communication port 46. The system bus 45 is configured to realize communication between components. The communication port 46 is used for communication between the electronic equipment and other peripherals.

The system bus mentioned in Fig. 4 may be a peripheral component interconnect (PCI) bus or an extended industry standard architecture (EISA) bus, etc. The system bus may be divided into an address bus, a data bus and a control bus. For convenience of representation, only a thick line is shown in the figure, but it does not mean that there is only one bus or one type of bus. The communication interface is configured to realize communication between a database access device and other devices (such as client, read-write library and read-only library). The memory may comprise a random access memory (RAM) or a non-volatile memory, such as at least one disk memory.

The above processor may be a general purpose processor, including a CPU, a network processor (NP), a graphics processing unit (GPU), etc., and may also be a DSP, an ASIC, an FPGA or other programmable logic devices, discrete gate or transistor logic devices, and discrete hardware components.

It should be noted that the embodiment of the invention also provides a computer-readable storage medium, in which instructions are stored, which, when run on a computer, cause the computer to execute the method and processing procedures provided in the above embodiments.

An embodiment of the invention also provides a chip for running instructions, and the chip is configured to execute the method and processing procedures provided in the above embodiments.

An embodiment of the invention also provides a program product, which comprises a computer program stored in a storage medium. At least one processor may read the computer program from the storage medium, and the at least one processor executes the method and processing procedures provided in the above embodiments.

The embodiment of the invention provides a blood pressure prediction method and device fusing nominal PPG signal data, electronic equipment, a computer program product and a computer-readable storage medium. Firstly, the nominal PPG signal data and corresponding nominal blood pressure data (nominal diastolic blood pressure data and nominal systolic blood pressure data) of a test object are obtained, then a well-trained CNN+ANN artificial intelligence blood pressure prediction network for predicting relative blood pressure data is used to perform blood pressure prediction operation on the fused data of the real-time PPG signal data and the nominal PPG signal of the test object to obtain relative blood pressure data (comprising relative diastolic blood pressure data and relative systolic blood pressure data), and then absolute blood pressure data calculation is performed on the nominal blood pressure data and the relative blood pressure data according to relative relationship information to obtain the final predicted blood pressure data. In this way, the prediction accuracy of an artificial intelligence blood pressure prediction network is improved.

Professionals should further realize that the units and algorithm steps of each example described in connection with the embodiments disclosed herein can be implemented in electronic hardware, computer software or a combination of the two. In order to clearly explain the interchangeability of hardware and software, the components and steps of each example have been generally described according to functions in the above description. Whether these functions are implemented in hardware or software depends on the specific application and design constraints of the technical scheme.

The steps of a method or algorithm described in connection with the embodiments disclosed herein may be implemented in hardware, a software module executed by a processor, or a combination of the two. The software module can be placed in a random access memory (RAM), memory, read-only memory (ROM), electrically programmable ROM, electrically erasable programmable ROM, register, hard disk, removable magnetic disk, CD-ROM, or any other form of storage medium known in the technical field.

The above-mentioned specific embodiments further explain the purpose, technical scheme and beneficial effects of the invention in detail. It should be understood that the above are only specific embodiments of the invention and are not used to limit the scope of protection of the invention.

## Claims

1. A blood pressure prediction method by fusing nominal photoplethysmography (PPG) signal data, which is a computer-implemented method, comprising:
acquiring the nominal PPG signal data and the corresponding nominal diastolic blood pressure data and nominal systolic blood pressure data;
acquiring real-time PPG signal data, a time length of the real-time PPG signal data being the same as that of the nominal PPG signal data;
performing input data preparation processing on a convolutional neural network (CNN) model according to the nominal PPG signal data and the real-time PPG signal data to generate a CNN input four-dimensional tensor;
using the CNN model to perform multi-layer convolution pooling calculation on the CNN input four-dimensional tensor to generate a CNN output four-dimensional tensor;
performing input data preparation processing on an artificial neural network (ANN) model according to the CNN output four-dimensional tensor to generate an ANN input two-dimensional tensor;
using the ANN model to perform regression calculation on the ANN input two-dimensional tensor to generate an ANN output two-dimensional tensor;
performing blood pressure data calculation according to the nominal diastolic blood pressure data, the nominal systolic blood pressure data and the ANN output two-dimensional tensor to generate a blood pressure two-dimensional tensor; and
when preset prediction type information is a first type, performing mean blood pressure data calculation according to the blood pressure two-dimensional tensor to generate diastolic blood pressure prediction data and systolic blood pressure prediction data, and when prediction type information is a second type, performing blood pressure data extraction processing on the blood pressure two-dimensional tensor to generate a diastolic blood pressure prediction data sequence and a systolic blood pressure prediction data sequence,
wherein performing input data preparation processing on a CNN model according to the nominal PPG signal data and the real-time PPG signal data to generate a CNN input four-dimensional tensor specifically comprises:
according to a preset sampling frequency, performing signal data sampling processing on the real-time PPG signal data to generate a real-time PPG data sequence, and performing signal data sampling processing on the nominal PPG signal data to generate a nominal PPG data sequence, a data length L₁ of the real-time PPG data sequence is the same as a data length L₂ of the nominal PPG data sequence;
according to a preset slot length, performing data slot division processing on the real-time PPG data sequence in sequence to obtain real-time PPG one-dimensional tensors, the number of which is the total number of real-time slots, and then fusing the real-time PPG one-dimensional tensors the number of which is the total number of real-time slots into one two-dimensional tensor to generate a real-time PPG two-dimensional tensor,
wherein the total number of real-time slots= int(L₁/ slot length), int () is a rounding function, the shape of the real-time PPG one-dimensional tensor is 1×W₁, the W₁ is a one-dimensional parameter of the real-time PPG one-dimensional tensor, and W₁= slot length, the shape of the real-time PPG two-dimensional tensor is B₁×W₂, the B₁ is a two-dimensional parameter of the real-time PPG two-dimensional tensor, and B₁= the total number of real-time slots, the W₂ is a one-dimensional parameter of the real-time PPG two-dimensional tensor, and W₂=W₁;
according to the slot length, performing data slot division processing on the nominal PPG data sequence in sequence to obtain nominal PPG one-dimensional tensors, the number of which is the total number of nominal slots, and then fusing the nominal PPG one-dimensional tensors the number of which is the total number of nominal slots into one two-dimensional tensor to generate a nominal PPG two-dimensional tensor,
wherein the total number of nominal slots= int(L₂/ slot length), the shape of the nominal PPG one-dimensional tensor is 1×W₃, the W₃ is a one-dimensional parameter of the nominal PPG one-dimensional tensor, and W₃= slot length, the shape of the nominal PPG two-dimensional tensor is B₂×W₄, the B₂ is a two-dimensional parameter of the nominal PPG two-dimensional tensor, and B₂= the total number of nominal slots, the W₄ is a one-dimensional parameter of the nominal PPG two-dimensional tensor, and W₄=W₃;
performing two-dimensional tensor fusion processing on the real-time PPG two-dimensional tensor and the nominal PPG two-dimensional tensor according to the sequence of nominal data following real-time data to generate a fused two-dimensional tensor,
wherein the shape of the fused two-dimensional tensor is B₃×W₅, the B₃ is a two-dimensional parameter of the fused two-dimensional tensor, and B₃=B₂=B₁, the W₅ is a one-dimensional parameter of the fused two-dimensional tensor, and W₅=W₂+W₄, the fused two-dimensional tensor comprises the B₃ fused one-dimensional tensors, and the fused one-dimensional tensor is formed by splicing the corresponding real-time PPG one-dimensional tensor and the nominal PPG one-dimensional tensor according to the sequence of the nominal PPG one-dimensional tensor following the real-time PPG one-dimensional tensor; and
according to a four-dimensional tensor input data format of the CNN model, raising the shape of the fused two-dimensional tensor from a two-dimensional tensor shape to a four-dimensional tensor shape to generate the CNN input four-dimensional tensor,
wherein the shape of the CNN input four-dimensional tensor is B₄×H₁×W₆×C₁, the B₄ is a four-dimensional parameter of the CNN input four-dimensional tensor, and B₄=B₃, the H₁ is a three-dimensional parameter of the CNN input four-dimensional tensor, and H₁=2, the W₆ is a two-dimensional parameter of the CNN input four-dimensional tensor, and W₆=W₅/2, the C₁ is a one-dimensional parameter of the CNN input four-dimensional tensor, and C₁=1,
wherein using the CNN model to perform multi-layer convolution pooling calculation on the CNN input four-dimensional tensor to generate a CNN output four-dimensional tensor specifically comprises:
taking the CNN input four-dimensional tensor as a first input four-dimensional tensor, and then sending the first input four-dimensional tensor to a first convolutional network layer of the CNN model for first-layer convolution pooling calculation to generate a first output four-dimensional tensor; then, taking the first output four-dimensional tensor as a second input four-dimensional tensor, and sending the second input four-dimensional tensor to a second convolutional network layer of the CNN model for second-layer convolution pooling calculation to generate a second output four-dimensional tensor; and finally, taking a penultimate output four-dimensional tensor as a last input four-dimensional tensor, and sending the last input four-dimensional tensor to a last convolutional network layer of the CNN model for last-layer convolution pooling calculation to generate the CNN output four-dimensional tensor,
wherein the CNN model comprises a plurality of convolutional network layers, the convolutional network layer comprises a convolution layer and a pooling layer, the shape of the CNN output four-dimensional tensor is B₅×H₂×W₇×C₂, the B₅ is a four-dimensional parameter of the CNN output four-dimensional tensor, and B₅=B₄, the H₂ is a three-dimensional parameter of the CNN output four-dimensional tensor, the W₇ is a two-dimensional parameter of the CNN output four-dimensional tensor, and the C₂ is a one-dimensional parameter of the CNN output four-dimensional tensor,
wherein performing input data preparation processing on an ANN model according to the CNN output four-dimensional tensor to generate an ANN input two-dimensional tensor specifically comprises:
according to a two-dimensional tensor input data format of the ANN model, reducing the shape of the CNN output four-dimensional tensor from a four-dimensional tensor shape to a two-dimensional tensor shape to generate the ANN input two-dimensional tensor,
wherein the shape of the ANN input two-dimensional tensor is B₆×W'₁, the B₆ is a two-dimensional parameter of the ANN input two-dimensional tensor, and B₆=B₅, the W'₁ is a one-dimensional parameter of the ANN input two-dimensional tensor, and W'₁=H₂*W₇*C₂, the ANN input two-dimensional tensor comprises the B₆ ANN input one-dimensional tensors, the shape of the ANN input one-dimensional tensor is 1×W'₂, the W'₂ is a one-dimensional parameter of the ANN input one-dimensional tensor, and W'₂= W'₁; and
adding the nominal diastolic blood pressure data and the nominal systolic blood pressure data to the end of each ANN input one-dimensional tensor, wherein the shape of the ANN input one-dimensional tensor becomes 1×W'₃, the W'₃ is a one-dimensional parameter of the ANN input one-dimensional tensor, and W'₃=W'₂+2=W'₁+2=H₂*W₇*C₂+2, the shape of the ANN input two-dimensional tensor become B₆×W₈, the W₈ is a new one-dimensional parameter of the ANN input two-dimensional tensor, and W₈=W'₃=H₂*W₇*C₂+2,
wherein using the ANN model to perform regression calculation on the ANN input two-dimensional tensor to generate an ANN output two-dimensional tensor specifically comprises:
taking the ANN input two-dimensional tensor as a first input two-dimensional tensor, and then sending the first input two-dimensional tensor to a first fully connected layer of the ANN model for first-layer full connection calculation to generate a first output two-dimensional tensor; then, taking the first output two-dimensional tensor as a second input two-dimensional tensor, and sending the second input two-dimensional tensor to a second fully connected layer of the ANN model for second-layer full connection calculation to generate a second output two-dimensional tensor; and finally, taking a penultimate output two-dimensional tensor as a last input two-dimensional tensor, and sending the last input two-dimensional tensor to a last fully connected layer of the ANN model for last-layer full connection calculation to generate the ANN output two-dimensional tensor,
wherein the ANN model comprises a plurality of fully connected layers, the shape of the ANN output two-dimensional tensor is B₇×W₉, the B₇ is a two-dimensional parameter of the ANN output two-dimensional tensor, and B₇=B₆, the W₉ is a one-dimensional parameter of the ANN output two-dimensional tensor, and W₉=2, the ANN output two-dimensional tensor comprises the B₇ ANN output one-dimensional tensors, and the ANN output one-dimensional tensor comprises relative diastolic blood pressure data and relative systolic blood pressure data,
wherein performing blood pressure data calculation according to the nominal diastolic blood pressure data, the nominal systolic blood pressure data and the ANN output two-dimensional tensor to generate a blood pressure two-dimensional tensor specifically comprises:
when the preset relative relation information is a difference relation, using the nominal diastolic blood pressure data to perform diastolic blood pressure value increase processing on all the relative diastolic blood pressure data in the ANN output two-dimensional tensor, using the nominal systolic blood pressure data to perform systolic blood pressure value increase processing on all the relative systolic blood pressure data in the ANN output two-dimensional tensor, and then, taking the ANN output two-dimensional tensor after the pressure value increasing process as the blood pressure two-dimensional tensor,
wherein the shape of the blood pressure two-dimensional tensor is B₈×W₁₀, the B₈ is a two-dimensional parameter of the blood pressure two-dimensional tensor, and B₈=B₇, the W₁₀ is a one-dimensional parameter of the blood pressure two-dimensional tensor, and W₁₀=2, the blood pressure two-dimensional tensor comprises the B₈ blood pressure one-dimensional tensors, the blood pressure one-dimensional tensor comprises diastolic blood pressure data and systolic blood pressure data, the diastolic blood pressure data is the sum of the corresponding relative diastolic blood pressure data and the nominal diastolic blood pressure data, and the systolic blood pressure data is the sum of the corresponding relative systolic blood pressure data and the nominal systolic blood pressure data,
wherein when preset prediction type information is a first type, performing mean blood pressure data calculation according to the blood pressure two-dimensional tensor to generate diastolic blood pressure prediction data and systolic blood pressure prediction data, and when prediction type information is a second type, performing blood pressure data extraction processing on the blood pressure two-dimensional tensor to generate a diastolic pressure prediction data sequence and a systolic pressure prediction data sequence specifically comprise:
when the prediction type information is the first type, calculating a mean value of all the diastolic blood pressure data in the blood pressure two-dimensional tensor to generate the diastolic blood pressure prediction data, and calculating a mean value of all the systolic blood pressure data in the blood pressure two-dimensional tensor to generate the systolic blood pressure prediction data; and
when the prediction type information is the second type, sequentially extracting the diastolic blood pressure data from the blood pressure two-dimensional tensor to form the diastolic blood pressure prediction data sequence, and extracting the systolic blood pressure data from the blood pressure two-dimensional tensor to form the systolic blood pressure prediction data sequence.

2. The blood pressure prediction method by fusing nominal PPG signal data according to claim 1, wherein sending the first input four-dimensional tensor to a first convolutional network layer of the CNN model for first-layer convolution pooling calculation to generate a first output four-dimensional tensor specifically comprises:
sending the first input four-dimensional tensor into a first convolution layer of the first convolutional network layer for first convolution calculation to generate a first convolution four-dimensional tensor, and sending the first convolution four-dimensional tensor into a first pooling layer of the first convolutional network layer for first pooling calculation to generate the first output four-dimensional tensor.

3. An electronic equipment, comprising a memory (42), a processor (41) and a transceiver (43),
wherein the processor (41) is configured to be coupled with the memory (42), and read and execute instructions in the memory (42), so as to implement the method according to any one of claims 1-2,
the transceiver (43) is coupled with the processor (41), and the processor (41) controls the transceiver (43) to send and receive messages.

4. A computer program product comprising a computer program code that, when executed by a computer, causes the computer to execute the method according to any one of claims 1-2.

5. A computer-readable storage medium, which stores computer instructions that, when executed by a computer, cause the computer to execute the method according to any one of claims 1-2.

## Patentansprüche

1. Verfahren zur Blutdruckvorhersage durch Zusammenführung von nominalen Photoplethysmographie-(PPG)-Signalen, wobei es sich um ein computerimplementiertes Verfahren handelt, umfassend:
Erfassen der nominalen PPG-Signale sowie der entsprechenden nominalen diastolischen Blutdruckdaten und nominalen systolischen Blutdruckdaten;
Erfassen von Echtzeit-PPG-Signalen, wobei die Zeitdauer der Echtzeit-PPG-Signale derjenigen der nominalen PPG-Signale entspricht;
Durchführung einer Eingabedaten-Aufbereitungsverarbeitung an einem Convolutional-Neural-Network-Modell (CNN-Modell) gemäß den nominalen PPG-Signalen und den Echtzeit-PPG-Signalen, um einen vierdimensionalen CNN-Eingabetensor zu erzeugen;
Verwendung des CNN-Modells zur Durchführung einer mehrschichtigen Faltungs-Pooling-Berechnung auf dem vierdimensionalen CNN-Eingabetensor, um einen vierdimensionalen CNN-Ausgabetensor zu erzeugen;
Durchführung einer Eingabedaten-Aufbereitungsverarbeitung an einem Modell eines künstlichen neuronalen Netzes (ANN) gemäß dem vierdimensionalen Ausgabetensor des CNN, um einen zweidimensionalen ANN-Eingabetensor zu erzeugen;
Verwendung des ANN-Modells zur Durchführung einer Regressionsberechnung am zweidimensionalen ANN-Eingabetensor, um einen zweidimensionalen ANN-Ausgabetensor zu erzeugen;
Durchführung einer Blutdruckdatenberechnung auf der Grundlage der nominalen diastolischen Blutdruckdaten, der nominalen systolischen Blutdruckdaten und des zweidimensionalen ANN-Ausgabetensors, um einen zweidimensionalen Blutdrucktensor zu erzeugen; und
wenn die voreingestellte Vorhersagetyp-Information einem ersten Typ entspricht, Durchschnittsblutdruckdatenberechnung gemäß dem zweidimensionalen Blutdrucktensor durchführen, um diastolische Blutdruck-Vorhersagedaten und systolische Blutdruck-Vorhersagedaten zu erzeugen, und wenn die Vorhersagetyp-Information einem zweiten Typ entspricht, eine Blutdruckdaten-Extraktionsverarbeitung am zweidimensionalen Blutdrucktensor durchführen, um eine diastolische Blutdruck-Vorhersagedatensequenz und eine systolische Blutdruck-Vorhersagedatensequenz zu erzeugen,
wobei das Durchführen einer Eingabedaten-Aufbereitungsverarbeitung an einem CNN-Modell gemäß den nominalen PPG-Signalen und den Echtzeit-PPG-Signalen zur Erzeugung des vierdimensionalen CNN-Eingabetensors insbesondere umfasst:
Durchführung einer Signaldaten-Abtastverarbeitung an den Echtzeit-PPG-Signalen gemäß einer voreingestellten Abtastfrequenz, um eine Echtzeit-PPG-Sequenz zu erzeugen, sowie Durchführung einer Signaldaten-Abtastverarbeitung an den nominalen PPG-Signalen, um eine nominale PPG-Sequenz zu erzeugen, wobei die Datenlänge L₁ der Echtzeit-PPG-Sequenz mit der Datenlänge L₂ der nominalen PPG-Sequenz übereinstimmt;
Durchführung einer Datenslot-Aufteilung der Echtzeit-PPG-Sequenz nacheinander gemäß einer voreingestellten Slot-Länge, um eindimensionale Echtzeit-PPG-Tensoren zu erhalten, deren Anzahl der Gesamtzahl der Echtzeit-Slots entspricht, und anschließendes Zusammenführen der eindimensionalen Echtzeit-PPG-Tensoren, deren Anzahl der Gesamtzahl der Echtzeit-Slots entspricht, zu einem zweidimensionalen Tensor, um einen zweidimensionalen Echtzeit-PPG-Tensor zu erzeugen,
wobei die Gesamtzahl der Echtzeit-Slots = int(L₁ / Slotlänge) ist, int () eine Rundungsfunktion ist, die Form des eindimensionalen Echtzeit-PPG-Tensors 1×W₁ ist, W₁ ein eindimensionaler Parameter des eindimensionalen Echtzeit-PPG-Tensors ist und W₁= Slotlänge ist, die Form des zweidimensionalen Echtzeit-PPG-Tensors B₁×W₂ ist, wobei B₁ ein zweidimensionaler Parameter des zweidimensionalen Echtzeit-PPG-Tensors ist und B₁= die Gesamtzahl der Echtzeit-Slots ist, W₂ ein eindimensionaler Parameter des zweidimensionalen Echtzeit-PPG-Tensors ist und W₂=W₁;
entsprechend der Slot-Länge wird nacheinander eine Datenslot-Aufteilungsverarbeitung an der nominalen PPG-Sequenz durchgeführt, um nominale PPG-eindimensionale Tensoren zu erhalten, deren Anzahl der Gesamtzahl der nominalen Slots entspricht, und anschließend werden die nominalen PPG-eindimensionalen Tensoren, deren Anzahl der Gesamtzahl der nominalen Slots entspricht, zu einem zweidimensionalen Tensor zusammengeführt, um einen nominalen PPG-zweidimensionalen Tensor zu erzeugen,
wobei die Gesamtzahl der nominalen Slots = int(L₂ / Slotlänge) ist, die Form des nominalen eindimensionalen PPG-Tensors 1×W₃ ist, wobei W₃ ein eindimensionaler Parameter des nominalen eindimensionalen PPG-Tensors ist und W₃= Slotlänge ist, die Form des nominalen zweidimensionalen PPG-Tensors B₂×W₄ ist, wobei B₂ ein zweidimensionaler Parameter des nominalen zweidimensionalen PPG-Tensors ist und B₂= der Gesamtzahl der nominalen Slots entspricht, W₄ ein eindimensionaler Parameter des nominalen zweidimensionalen PPG-Tensors ist und W₄=W₃ ist;
Durchführung einer zweidimensionalen Tensor-Zusammenführung des Echtzeit-PPG-zweidimensionalen Tensors und des nominalen PPG-zweidimensionalen Tensors gemäß der Sequenz der nominalen Daten, die auf die Echtzeitdaten folgen, um einen fusionierten zweidimensionalen Tensor zu erzeugen,
wobei die Form des fusionierten zweidimensionalen Tensors B₃×W₅ ist, wobei B₃ ein zweidimensionaler Parameter des fusionierten zweidimensionalen Tensors ist und B₃=B₂=B₁ gilt, W₅ ein eindimensionaler Parameter des fusionierten zweidimensionalen Tensors ist und W₅=W₂+W₄ ist, der fusionierte zweidimensionale Tensor die B₃ fusionierten eindimensionalen Tensoren umfasst und der fusionierte eindimensionale Tensor durch Zusammenfügen des entsprechenden Echtzeit-PPG-eindimensionalen Tensors und des nominalen PPG-eindimensionalen Tensors gemäß der Sequenz gebildet wird, bei der der nominale PPG-eindimensionale Tensor auf den Echtzeit-PPG-eindimensionalen Tensor folgt; und
gemäß einem vierdimensionalen Tensor-Eingabedatenformat des CNN-Modells wird die Form des fusionierten zweidimensionalen Tensors von einer zweidimensionalen Tensorform auf eine vierdimensionale Tensorform erweitert, um den vierdimensionalen CNN-Eingabetensor zu erzeugen,
wobei die Form des vierdimensionalen CNN-Eingabetensors B₄×H₁×W₆×C₁ ist, B₄ ein vierdimensionaler Parameter des vierdimensionalen CNN-Eingabetensors ist und B₄=B₃ ist, H₁ ein dreidimensionaler Parameter des vierdimensionalen CNN-Eingabetensors ist und H₁=2 ist, W₆ ein zweidimensionaler Parameter des vierdimensionalen CNN-Eingabetensors ist und W₆=W₅/2, C₁ ist ein eindimensionaler Parameter des vierdimensionalen CNN-Eingabetensors und C₁=1,
wobei die Verwendung des CNN-Modells zur Durchführung einer mehrschichtigen Faltungs-Pooling-Berechnung auf dem vierdimensionalen CNN-Eingabetensor zur Erzeugung eines vierdimensionalen CNN-Ausgabetensors konkret Folgendes umfasst:
den vierdimensionalen CNN-Eingabetensor als ersten vierdimensionalen Eingabetensor zu nehmen und anschließend den ersten vierdimensionalen Eingabetensor an eine erste Faltungsnetzwerkschicht des CNN-Modells zu senden, um eine Faltungs-Pooling-Berechnung der ersten Schicht durchzuführen und einen ersten vierdimensionalen Ausgabetensor zu erzeugen; anschließend den ersten vierdimensionalen Ausgabetensor als zweiten vierdimensionalen Eingabetensor zu verwenden und den zweiten vierdimensionalen Eingabetensor an eine zweite Faltungsnetzwerkschicht des CNN-Modells zu leiten, um dort die Faltungs-Pooling-Berechnung der zweiten Schicht durchzuführen und einen zweiten vierdimensionalen Ausgabetensor zu erzeugen; und schließlich wird ein vorletzter vierdimensionaler Ausgabetensor als letzter vierdimensionaler Eingabetensor verwendet und der letzte vierdimensionale Eingabetensor an eine letzte Faltungsnetzwerkschicht des CNN-Modells gesendet, um eine Faltungs-Pooling-Berechnung der letzten Schicht durchzuführen und den vierdimensionalen CNN-Ausgabetensor zu erzeugen,
wobei das CNN-Modell eine Vielzahl von Faltungsnetzwerkschichten umfasst, die Faltungsnetzwerkschicht eine Faltungsschicht und eine Pooling-Schicht umfasst, die Form des vierdimensionalen CNN-Ausgabetensors B₅×H₂×W₇×C₂ ist, B₅ ein vierdimensionaler Parameter des vierdimensionalen Ausgabetensors ist und B₅=B₄ ist, H₂ ein dreidimensionaler Parameter des vierdimensionalen Ausgabetensors des CNN ist, W₇ ein zweidimensionaler Parameter des vierdimensionalen Ausgabetensors des CNN ist und C₂ ein eindimensionaler Parameter des vierdimensionalen Ausgabetensors des CNN ist,
wobei die Aufbereitung der Eingabedaten für ein ANN-Modell gemäß dem vierdimensionalen Ausgabetensor des CNN, um einen zweidimensionalen ANN-Eingabetensor zu erzeugen, insbesondere Folgendes umfasst:
Entsprechend einem zweidimensionalen Tensor-Eingabedatenformat des ANN-Modells die Form des vierdimensionalen CNN-Ausgabetensors von einer vierdimensionalen Tensorform auf eine zweidimensionale Tensorform zu reduzieren, um den zweidimensionalen ANN-Eingabetensor zu erzeugen,
wobei die Form des zweidimensionalen ANN-Eingabetensors B₆×W'₁ ist, wobei B₆ ein zweidimensionaler Parameter des zweidimensionalen ANN-Eingabetensors ist und B₆=B₅ gilt, W'₁ein eindimensionaler Parameter des zweidimensionalen ANN-Eingabetensors ist und W'₁=H₂*W₇*C₂ ist, der zweidimensionale ANN-Eingabetensor die B₆ eindimensionalen ANN-Eingabetensoren umfasst, die Form des eindimensionalen ANN-Eingabetensors 1×W'₂ ist, W'₂ ein eindimensionaler Parameter des eindimensionalen ANN-Eingabetensors ist und W'₂=W'₁; und
Hinzufügen der nominalen diastolischen Blutdruckdaten und der nominalen systolischen Blutdruckdaten an das Ende jedes eindimensionalen ANN-Eingabetensors, wobei die Form des eindimensionalen ANN-Eingabetensors zu 1×W'₃ wird, wobei W'₃ ein eindimensionaler Parameter des eindimensionalen ANN-Eingabetensors ist und W'₃=W'₂+2=W'₁+2=H₂*W₇*C₂+2 gilt; die Form des zweidimensionalen ANN-Eingabetensors wird zu B₆×W₈ , wobei W₈ ein neuer eindimensionaler Parameter des zweidimensionalen ANN-Eingabetensors ist und W₈=W'₃=H₂*W₇*C₂+2,
wobei die Durchführung einer Regressionsberechnung am zweidimensionalen Eingabetensor des ANN unter Verwendung des ANN-Modells zur Erzeugung eines zweidimensionalen Ausgabetensors des ANN konkret Folgendes umfasst:
den zweidimensionalen ANN-Eingabetensor als ersten zweidimensionalen Eingabetensor zu nehmen und anschließend den ersten zweidimensionalen Eingabetensor an eine erste vollständig verbundene Schicht des ANN-Modells zu senden, um eine Berechnung der ersten Schicht mit vollständiger Verbindung durchzuführen und einen ersten zweidimensionalen Ausgabetensor zu erzeugen; anschließend den ersten zweidimensionalen Ausgabetensor als zweiten zweidimensionalen Eingabetensor zu verwenden und den zweiten zweidimensionalen Eingabetensor an eine zweite vollständig verbundene Schicht des ANN-Modells zu senden, um eine Berechnung der vollständigen Verbindung der zweiten Schicht durchzuführen und einen zweiten zweidimensionalen Ausgabetensor zu erzeugen; und schließlich wird ein vorletzter zweidimensionaler Ausgabetensor als letzter zweidimensionaler Eingabetensor verwendet und der letzte zweidimensionale Eingabetensor an eine letzte vollständig verbundene Schicht des ANN-Modells weitergeleitet, um eine Berechnung der vollständigen Verbindung der letzten Schicht durchzuführen und den zweidimensionalen ANN-Ausgabetensor zu erzeugen,
wobei das ANN-Modell eine Vielzahl von vollständig verbundenen Schichten umfasst, die Form des zweidimensionalen ANN-Ausgabetensors B₇×W₉ ist, B₇ ein zweidimensionaler Parameter des zweidimensionalen ANN-Ausgabetensors ist und B₇=B₆ ist, W₉ ein eindimensionaler Parameter des zweidimensionalen ANN-Ausgabetensors ist und W₉=2 ist, der zweidimensionale ANN-Ausgabetensor die B₇ eindimensionalen ANN-Ausgabetensoren umfasst und der eindimensionale ANN-Ausgabetensor relative diastolische Blutdruckdaten und relative systolische Blutdruckdaten umfasst,
wobei die Durchführung einer Blutdruckdatenberechnung gemäß den nominalen diastolischen Blutdruckdaten, den nominalen systolischen Blutdruckdaten und dem zweidimensionalen Tensor der ANN-Ausgabe zur Erzeugung eines zweidimensionalen Blutdrucktensors insbesondere Folgendes umfasst:
wenn die voreingestellte relative Beziehungsinformation eine Differenzbeziehung ist, die Verwendung der nominalen diastolischen Blutdruckdaten, um eine Erhöhung der diastolischen Blutdruckwerte für alle relativen diastolischen Blutdruckdaten im zweidimensionalen Ausgabetensor der ANN-Ausgabe durchzuführen, die Verwendung der nominalen systolischen Blutdruckdaten, um eine Erhöhung der systolischen Blutdruckwerte für alle relativen systolischen Blutdruckdaten im zweidimensionalen Ausgabetensor der ANN-Ausgabe durchzuführen, und anschließend den zweidimensionalen Tensor der ANN-Ausgabe nach dem Prozess der Erhöhung der Druckwerte als den zweidimensionalen Blutdrucktensor zu verwenden,
wobei die Form des zweidimensionalen Blutdrucktensors B₈×W₁₀ ist, wobei B₈ ein zweidimensionaler Parameter des zweidimensionalen Blutdrucktensors ist und B₈=B₇ ist, W₁₀ ein eindimensionaler Parameter des zweidimensionalen Blutdrucktensors ist und W₁₀=2 ist, der zweidimensionale Blutdrucktensor die B₈ eindimensionale Blutdrucktensoren, der eindimensionale Blutdrucktensor umfasst diastolische Blutdruckdaten und systolische Blutdruckdaten, die diastolischen Blutdruckdaten sind die Summe der entsprechenden relativen diastolischen Blutdruckdaten und der nominalen diastolischen Blutdruckdaten, und die systolischen Blutdruckdaten sind die Summe der entsprechenden relativen systolischen Blutdruckdaten und der nominalen systolischen Blutdruckdaten,
wobei, wenn die voreingestellte Vorhersagetyp-Information einem ersten Typ entspricht, eine Durchschnittsblutdruckdatenberechnung gemäß dem zweidimensionalen Blutdrucktensor durchgeführt wird, um diastolische Blutdruck-Vorhersagedaten und systolische Blutdruck-Vorhersagedaten zu erzeugen, und wenn die Vorhersagetyp-Information einem zweiten Typ entspricht, eine Blutdruckdaten-Extraktionsverarbeitung am zweidimensionalen Blutdrucktensor durchgeführt wird, um eine diastolische Blutdruck-Vorhersagedatensequenz und eine systolische Blutdruck-Vorhersagedatensequenz zu erzeugen, die insbesondere Folgendes umfassen:
wenn die Vorhersagetyp-Information den ersten Typ aufweist, Berechnen eines Mittelwerts aller diastolischen Blutdruckdaten im zweidimensionalen Blutdrucktensor, um die diastolischen Blutdruck-Vorhersagedaten zu erzeugen, und Berechnen eines Mittelwerts aller systolischen Blutdruckdaten im zweidimensionalen Blutdrucktensor, um die systolischen Blutdruck-Vorhersagedaten zu erzeugen; und
wenn die Vorhersagetyp-Information den zweiten Typ darstellt, das sequenzielle Extrahieren der diastolischen Blutdruckdaten aus dem zweidimensionalen Blutdrucktensor, um die diastolische Blutdruck-Vorhersagedatensequenz zu bilden, und das Extrahieren der systolischen Blutdruckdaten aus dem zweidimensionalen Blutdrucktensor, um die systolische Blutdruck-Vorhersagedatensequenz zu bilden.

2. Verfahren zur Blutdruckvorhersage durch Zusammenführung von nominalen PPG-Signalen gemäß Anspruch 1, wobei das Senden des ersten vierdimensionalen Eingabetensors an eine erste Faltungsnetzwerkschicht des CNN-Modells zur Berechnung des Faltungs-Poolings der ersten Schicht zur Erzeugung eines ersten vierdimensionalen Ausgabetensors insbesondere umfasst:
das Senden des ersten vierdimensionalen Eingabetensors in eine erste Faltungsschicht des ersten Faltungsnetzwerks zur Durchführung einer ersten Faltungsberechnung, um einen ersten vierdimensionalen Faltungstensor zu erzeugen, und das Senden des ersten vierdimensionalen Faltungstensors in eine erste Pooling-Schicht des ersten Faltungsnetzwerks zur Durchführung einer ersten Pooling-Berechnung, um den ersten vierdimensionalen Ausgabetensor zu erzeugen.

3. Elektronisches Gerät, umfassend einen Speicher (42), einen Prozessor (41) und einen Transceiver (43),
wobei der Prozessor (41) so konfiguriert ist, dass er mit dem Speicher (42) gekoppelt ist und Befehle aus dem Speicher (42) liest und ausführt, um das Verfahren gemäß einem der Ansprüche 1-2 durchzuführen,
der Transceiver (43) mit dem Prozessor (41) gekoppelt ist und der Prozessor (41) den Transceiver (43) so steuert, dass dieser Nachrichten sendet und empfängt.

4. Computerprogrammprodukt, das einen Computerprogrammcode umfasst, der, wenn er von einem Computer ausgeführt wird, den Computer veranlasst, das Verfahren gemäß einem der Ansprüche 1-2 auszuführen.

5. Computerlesbares Speichermedium, auf dem Computerbefehle gespeichert sind, die, wenn sie von einem Computer ausgeführt werden, den Computer veranlassen, das Verfahren gemäß einem der Ansprüche 1-2 auszuführen.

## Revendications

1. Procédé de prédiction de la pression artérielle par fusion de données de signal de photopléthysmographie (PPG) nominales, qui est un procédé mis en œuvre par ordinateur, comprenant:
l'acquisition des données de signal PPG nominales et des données correspondantes de pression artérielle diastolique nominale et de pression artérielle systolique nominale;
l'acquisition de données de signal PPG en temps réel, la durée des données de signal PPG en temps réel étant identique à celle des données de signal PPG nominales;
la réalisation d'un traitement de préparation des données d'entrée sur un modèle de réseau neuronal convolutif (CNN) en fonction des données de signal PPG nominales et des données de signal PPG en temps réel afin de générer un tenseur à quatre dimensions d'entrée du CNN;
utiliser le modèle CNN pour effectuer un calcul de convolution et de pooling multicouche sur le tenseur à quatre dimensions d'entrée du CNN afin de générer un tenseur à quatre dimensions de sortie du CNN;
effectuer un traitement de préparation des données d'entrée sur un modèle de réseau neuronal artificiel (ANN) en fonction du tenseur à quatre dimensions de sortie du CNN afin de générer un tenseur à deux dimensions d'entrée de l'ANN;
utiliser le modèle ANN pour effectuer un calcul de régression sur le tenseur bidimensionnel d'entrée de l'ANN afin de générer un tenseur bidimensionnel de sortie de l'ANN;
effectuer un calcul des données de pression artérielle en fonction des données nominales de pression artérielle diastolique, des données nominales de pression artérielle systolique et du tenseur bidimensionnel de sortie de l'ANN afin de générer un tenseur bidimensionnel de pression artérielle; et
lorsque les informations de type de prédiction prédéfinies correspondent à un premier type, effectuer un calcul des données de pression artérielle moyenne en fonction du tenseur bidimensionnel de pression artérielle afin de générer des données de prédiction de la pression artérielle diastolique et des données de prédiction de la pression artérielle systolique, et lorsque les informations de type de prédiction correspondent à un deuxième type, effectuer un traitement d'extraction des données de pression artérielle sur le tenseur bidimensionnel de pression artérielle afin de générer une séquence de données de prédiction de la pression artérielle diastolique et une séquence de données de prédiction de la pression artérielle systolique,
dans lequel l'exécution d'un traitement de préparation des données d'entrée sur un modèle CNN en fonction des données de signal PPG nominales et des données de signal PPG en temps réel afin de générer un tenseur d'entrée à quatre dimensions du modèle CNN comprend spécifiquement:
en fonction d'une fréquence d'échantillonnage prédéfinie, effectuer un traitement d'échantillonnage des données de signal sur les données de signal PPG en temps réel afin de générer une séquence de données PPG en temps réel, et effectuer un traitement d'échantillonnage des données de signal sur les données de signal PPG nominales afin de générer une séquence de données PPG nominales, la longueur de données L₁ de la séquence de données PPG en temps réel étant identique à la longueur de données L₂ de la séquence de données PPG nominales;
selon une longueur de créneau prédéfinie, effectuer un traitement de division en créneaux de données sur la séquence de données PPG en temps réel, de manière séquentielle, afin d'obtenir des tenseurs PPG unidimensionnels en temps réel, dont le nombre correspond au nombre total de créneaux en temps réel, puis fusionner les tenseurs unidimensionnels PPG en temps réel, dont le nombre correspond au nombre total de créneaux en temps réel, en un seul tenseur bidimensionnel afin de générer un tenseur PPG bidimensionnel en temps réel,
où le nombre total de créneaux en temps réel = int(L₁ / longueur de créneau), int () étant une fonction d'arrondi, la forme du tenseur unidimensionnel PPG en temps réel est 1×W₁, W₁ étant un paramètre unidimensionnel du tenseur unidimensionnel PPG en temps réel, et W₁= longueur de créneau, la forme du tenseur bidimensionnel PPG en temps réel est B₁×W₂, B₁ est un paramètre bidimensionnel du tenseur bidimensionnel PPG en temps réel, et B₁= nombre total de créneaux en temps réel, W₂ est un paramètre unidimensionnel du tenseur bidimensionnel PPG en temps réel, et W₂=W₁;
en fonction de la longueur des créneaux, effectuer successivement un traitement de division en créneaux de données sur la séquence de données PPG nominale afin d'obtenir des tenseurs PPG unidimensionnels nominaux, dont le nombre correspond au nombre total de créneaux nominaux, puis fusionner les tenseurs PPG unidimensionnels nominaux, dont le nombre correspond au nombre total de créneaux nominaux, en un seul tenseur bidimensionnel afin de générer un tenseur PPG bidimensionnel nominal,
où le nombre total de créneaux nominales = int(L₂/ longueur de créneau), la forme du tenseur unidimensionnel PPG nominal est 1×W₃, W₃ étant un paramètre unidimensionnel du tenseur unidimensionnel PPG nominal, et W₃= longueur de créneau, la forme du tenseur bidimensionnel PPG nominal est B₂×W₄, où B₂ est un paramètre bidimensionnel du tenseur PPG nominal bidimensionnel et B₂= le nombre total de créneaux nominaux, et W₄ est un paramètre unidimensionnel du tenseur PPG nominal bidimensionnel et W₄=W₃;
effectuer un traitement de fusion de tenseurs bidimensionnels sur le tenseur bidimensionnel PPG en temps réel et le tenseur bidimensionnel PPG nominal selon la séquence des données nominales suivant les données en temps réel afin de générer un tenseur bidimensionnel fusionné,
la forme du tenseur bidimensionnel fusionné étant B_{3×}W₅, où B₃ est un paramètre bidimensionnel du tenseur bidimensionnel fusionné et B₃=B₂=B₁, et W₅ est un paramètre unidimensionnel du tenseur bidimensionnel fusionné et W₅=W₂+W₄, le tenseur bidimensionnel fusionné comprend les B₃ tenseurs unidimensionnels fusionnés, et le tenseur unidimensionnel fusionné est formé par concaténation le tenseur unidimensionnel PPG en temps réel correspondant et le tenseur unidimensionnel PPG nominal selon la séquence du tenseur unidimensionnel PPG nominal suivant le tenseur unidimensionnel PPG en temps réel; et
conformément à un format de données d'entrée de tenseur à quatre dimensions du modèle CNN, en faisant passer la forme du tenseur bidimensionnel fusionné d'une forme de tenseur bidimensionnel à une forme de tenseur à quatre dimensions afin de générer le tenseur à quatre dimensions d'entrée du CNN,
où la forme du tenseur à quatre dimensions d'entrée du CNN est B₄×H₁×W₆×C₁, B₄ est un paramètre à quatre dimensions du tenseur à quatre dimensions d'entrée du CNN, et B₄=B₃, H₁ est un paramètre tridimensionnel du tenseur à quatre dimensions d'entrée du CNN, et H₁=2, W₆ est un paramètre bidimensionnel du tenseur à quatre dimensions d'entrée du CNN, et W₆=W₅/2, C₁ est un paramètre unidimensionnel du tenseur à quatre dimensions d'entrée du CNN, et C₁=1,
dans lequel l'utilisation du modèle CNN pour effectuer un calcul de convolution-pooling multicouche sur le tenseur à quatre dimensions d'entrée du CNN afin de générer un tenseur à quatre dimensions de sortie du CNN comprend spécifiquement:
en prenant le tenseur à quatre dimensions d'entrée du CNN comme premier tenseur à quatre dimensions d'entrée, puis en envoyant ce premier tenseur à quatre dimensions d'entrée à une première couche du réseau convolutif du modèle CNN pour effectuer le calcul de pooling par convolution de la première couche afin de générer un premier tenseur à quatre dimensions de sortie; ensuite, prendre le premier tenseur de sortie à quatre dimensions comme deuxième tenseur d'entrée à quatre dimensions, et envoyer ce deuxième tenseur d'entrée à quatre dimensions à une deuxième couche du réseau convolutif du modèle CNN afin d'effectuer le calcul de pooling par convolution de la deuxième couche pour générer un deuxième tenseur de sortie à quatre dimensions; et enfin, prendre un avant-dernier tenseur de sortie à quatre dimensions comme dernier tenseur d'entrée à quatre dimensions, et envoyer ce dernier tenseur d'entrée à quatre dimensions vers une dernière couche de réseau convolutif du modèle CNN afin d'effectuer le calcul de pooling par convolution de la dernière couche pour générer le tenseur de sortie à quatre dimensions du CNN,
le modèle CNN comprenant une pluralité de couches de réseau convolutif, chaque couche de réseau convolutif comprenant une couche de convolution et une couche de pooling, la forme du tenseur à quatre dimensions de sortie du CNN étant B₅×H₂×W₇×C₂, où B₅ est un paramètre à quatre dimensions du tenseur à quatre dimensions de sortie du CNN, et B₅=B₄, H₂ est un paramètre tridimensionnel du tenseur à quatre dimensions de sortie du CNN, W₇ est un paramètre bidimensionnel du tenseur à quatre dimensions de sortie du CNN, et C₂ est un paramètre unidimensionnel du tenseur à quatre dimensions de sortie du CNN,
dans lequel l'exécution du traitement de préparation des données d'entrée sur un modèle ANN selon le tenseur à quatre dimensions de sortie du CNN pour générer un tenseur à deux dimensions d'entrée de l'ANN, comprend spécifiquement:
conformément à un format de données d'entrée sous forme de tenseur bidimensionnel du modèle ANN, la réduction de la forme du tenseur à quatre dimensions de sortie du CNN, d'une forme de tenseur à quatre dimensions à une forme de tenseur bidimensionnel, afin de générer le tenseur bidimensionnel d'entrée de l'ANN,
la forme du tenseur bidimensionnel d'entrée du réseau neuronal artificiel étant B₆×W'₁, où B₆ est un paramètre bidimensionnel du tenseur bidimensionnel d'entrée du réseau neuronal artificiel et B₆=B₅, W'₁ est un paramètre unidimensionnel du tenseur bidimensionnel d'entrée du réseau neuronal artificiel, et W'₁=H₂*W₇*C₂, le tenseur bidimensionnel d'entrée du réseau neuronal artificiel (ANN) comprend les B₆ tenseurs unidimensionnels d'entrée de l'ANN, la forme du tenseur unidimensionnel d'entrée de l'ANN est 1×W'₂, W'₂ est un paramètre unidimensionnel du tenseur unidimensionnel d'entrée de l'ANN, et W'₂=W'₁; et
en ajoutant les données de pression artérielle diastolique nominale et les données de pression artérielle systolique nominale à la fin de chaque tenseur unidimensionnel d'entrée du réseau neuronal artificiel (ANN), la forme du tenseur unidimensionnel d'entrée de l'ANN devenant alors 1×W'₃, où W'₃ est un paramètre unidimensionnel du tenseur unidimensionnel d'entrée de l'ANN, et W'₃=W'₂+2=W'₁+2=H₂*W₇*C₂+2, la forme du tenseur bidimensionnel d'entrée du réseau neuronal artificiel (ANN) devient B₆×W₈, où W₈ est un nouveau paramètre unidimensionnel du tenseur bidimensionnel d'entrée du réseau neuronal artificiel (ANN), et W₈=W'₃=H₂*W₇*C₂+ 2,
dans lequel l'utilisation du modèle de réseau neuronal artificiel (ANN) pour effectuer un calcul de régression sur le tenseur bidimensionnel d'entrée de l'ANN afin de générer un tenseur bidimensionnel de sortie de l'ANN comprend spécifiquement:
prendre le tenseur bidimensionnel d'entrée du réseau neuronal artificiel (ANN) comme premier tenseur bidimensionnel d'entrée, puis envoyer ce premier tenseur bidimensionnel d'entrée à une première couche entièrement connectée du modèle ANN pour effectuer un calcul de connexion complète de première couche afin de générer un premier tenseur bidimensionnel de sortie; ensuite, prendre le premier tenseur bidimensionnel de sortie comme deuxième tenseur bidimensionnel d'entrée, et envoyer ce deuxième tenseur bidimensionnel d'entrée à une deuxième couche entièrement connectée du modèle ANN pour effectuer un calcul de connexion complète de la deuxième couche afin de générer un deuxième tenseur bidimensionnel de sortie; et enfin, prendre un avant-dernier tenseur bidimensionnel de sortie comme dernier tenseur bidimensionnel d'entrée, et envoyer ce dernier tenseur bidimensionnel d'entrée à une dernière couche entièrement connectée du modèle ANN pour effectuer le calcul de connexion complète de la dernière couche afin de générer le tenseur bidimensionnel de sortie de l'ANN,
le modèle ANN comprenant une pluralité de couches entièrement connectées, la forme du tenseur bidimensionnel de sortie de l'ANN étant B₇xW₉, B₇ étant un paramètre bidimensionnel du tenseur bidimensionnel de sortie de l'ANN, et B₇=B₆, W₉ est un paramètre unidimensionnel du tenseur bidimensionnel de sortie du réseau neuronal artificiel, et W₉=2, le tenseur bidimensionnel de sortie du réseau neuronal artificiel comprend les B₇ tenseurs unidimensionnels de sortie du réseau neuronal artificiel, et le tenseur unidimensionnel de sortie du réseau neuronal artificiel comprend des données de pression artérielle diastolique relative et des données de pression artérielle systolique relative,
dans lequel le calcul des données de pression artérielle, effectué en fonction des données nominales de pression artérielle diastolique, des données nominales de pression artérielle systolique et du tenseur bidimensionnel de sortie du réseau neuronal artificiel (ANN) afin de générer un tenseur bidimensionnel de pression artérielle, comprend spécifiquement:
lorsque les informations de relation relative prédéfinies correspondent à une relation de différence, l'utilisation des données de pression artérielle diastolique nominale pour effectuer un traitement d'augmentation de la valeur de la pression artérielle diastolique sur l'ensemble des données de pression artérielle diastolique relative contenues dans le tenseur bidimensionnel de sortie du réseau neuronal artificiel, l'utilisation des données de pression artérielle systolique nominale pour effectuer un traitement d'augmentation de la valeur de la pression artérielle systolique sur l'ensemble des données de pression artérielle systolique relative contenues dans le tenseur bidimensionnel de sortie du réseau neuronal artificiel, puis, considérer le tenseur bidimensionnel de sortie du réseau neuronal artificiel (ANN) après le processus d'augmentation des valeurs de pression comme le tenseur bidimensionnel de pression artérielle,
où la forme du tenseur bidimensionnel de la pression artérielle est B₈×W₁₀, B₈ étant un paramètre bidimensionnel du tenseur bidimensionnel de la pression artérielle, et B₈=B₇, W₁₀ est un paramètre unidimensionnel du tenseur bidimensionnel de la pression artérielle, et W₁₀=2, le tenseur bidimensionnel de la pression artérielle comprend les B8 tenseurs unidimensionnels de pression artérielle; le tenseur unidimensionnel de pression artérielle comprend des données de pression artérielle diastolique et des données de pression artérielle systolique; les données de pression artérielle diastolique correspondent à la somme des données de pression artérielle diastolique relative et des données de pression artérielle diastolique nominale; et les données de pression artérielle systolique correspondent à la somme des données de pression artérielle systolique relative et des données de pression artérielle systolique nominale,
dans lequel, lorsque les informations de type de prédiction prédéfinies correspondent à un premier type, effectuer un calcul de la moyenne des données de pression artérielle selon le tenseur bidimensionnel de pression artérielle afin de générer des données de prédiction de la pression artérielle diastolique et des données de prédiction de la pression artérielle systolique, et lorsque les informations de type de prédiction correspondent à un deuxième type, effectuer un traitement d'extraction des données de pression artérielle sur le tenseur bidimensionnel de pression artérielle afin de générer une séquence de données de prédiction de la pression diastolique et une séquence de données de prédiction de la pression systolique, comprenant spécifiquement:
lorsque les informations de type de prédiction correspondent au premier type, le calcul d'une valeur moyenne de toutes les données de pression artérielle diastolique dans le tenseur bidimensionnel de pression artérielle afin de générer les données de prédiction de la pression artérielle diastolique, et le calcul d'une valeur moyenne de toutes les données de pression artérielle systolique dans le tenseur bidimensionnel de pression artérielle afin de générer les données de prédiction de la pression artérielle systolique; et
lorsque les informations de type de prédiction correspondent au deuxième type, extraire séquentiellement les données de pression artérielle diastolique du tenseur bidimensionnel de pression artérielle pour former la séquence de données de prédiction de la pression artérielle diastolique, et extraire les données de pression artérielle systolique du tenseur bidimensionnel de pression artérielle pour former la séquence de données de prédiction de la pression artérielle systolique.

2. Procédé de prédiction de la pression artérielle par fusion de données de signal PPG nominales selon la revendication 1, dans lequel l'envoi du premier tenseur d'entrée à quatre dimensions vers une première couche de réseau convolutif du modèle CNN pour un calcul de pooling par convolution de première couche afin de générer un premier tenseur de sortie à quatre dimensions comprend spécifiquement:
l'envoi du premier tenseur d'entrée à quatre dimensions vers une première couche de convolution de la première couche du réseau convolutif afin d'effectuer un premier calcul de convolution pour générer un premier tenseur de convolution à quatre dimensions, et l'envoi du premier tenseur de convolution à quatre dimensions vers une première couche de pooling de la première couche du réseau convolutif afin d'effectuer un premier calcul de pooling pour générer le premier tenseur de sortie à quatre dimensions.

3. Équipement électronique, comprenant une mémoire (42), un processeur (41) et un émetteur-récepteur (43),
dans lequel le processeur (41) est configuré pour être couplé à la mémoire (42), et pour lire et exécuter des instructions contenues dans la mémoire (42), de manière à mettre en œuvre le procédé selon l'une quelconque des revendications 1 à 2,
dans lequel l'émetteur-récepteur (43) est couplé au processeur (41), et le processeur (41) commande l'émetteur-récepteur (43) pour envoyer et recevoir des messages.

4. Produit-programme d'ordinateur comprenant un code de programme informatique qui, lorsqu'il est exécuté par un ordinateur, amène celui-ci à exécuter le procédé selon l'une quelconque des revendications 1 à 2.

5. Support de stockage lisible par ordinateur, qui stocke des instructions informatiques qui, lorsqu'elles sont exécutées par un ordinateur, amènent celui-ci à exécuter le procédé selon l'une quelconque des revendications 1 à 2.
